# EUROPEAN PATENT SPECIFICATION

(11) **EP 4 003 259 B1**
(45) Date of publication and mention of the grant of the patent: **17.09.2025**
(21) Application number: 20753603.8
(22) Date of filing: 20.07.2020
(51) Int. Cl.: A61F 13/53, A61F 13/534, A61F 13/539, B32B 5/02, B32B 5/26

(54) **ABSORBENT LAMINATE INCLUDING A SPUNLACE NONWOVEN LAYER, ABSORBENT CORES WITH SUCH LAMINATES, AND ABSORBENT ARTICLES WITH SUCH ABSORBENT CORES**
ABSORBIERENDES LAMINAT MIT EINER SCHICHT AUS WASSERSTRAHLVERFESTIGTEM VLIES, ABSORBIERENDE KERNE MIT SOLCHEN LAMINATEN UND ABSORBIERENDE ARTIKEL MIT SOLCHEN ABSORBIERENDEN KERNEN
STRATIFIÉ ABSORBANT COMPRENANT UNE COUCHE DE NON-TISSÉ HYDROLIÉ, NOYAUX ABSORBANTS MUNI DE TELS STRATIFIÉS, ET ARTICLES ABSORBANTS MUNIS DE TELS NOYAUX ABSORBANTS

(30) Priority: 29.07.2019 US 201962879879 P
(43) Date of publication of application: 01.06.2022
(73) Proprietor: Attends Healthcare Products, Inc., Raleigh, NC 27617 (US)
(72) Inventor: CHMIELEWSKI, Harry, Raleigh, NC 27617 (US); DUCKER, Paul, Raleigh, NC 27617 (US); SCHROER, JR. Charles F., Raleigh, NC 27617 (US); ASHCRAFT, Matthew, Raleigh, NC 27617 (US); COSTELLO, John, Raleigh, NC 27617 (US)
(74) Representative: Lohr, Jöstingmeier & Partner Patent- und Rechtsanwälte mbB
(86) International application number: PCT/US2020/042720
(87) International publication number: WO 2021/021475

(56) References cited:
- WO-A1-2018/112229
- WO-A1-99/49826
- US-A1- 2004 024 375
- US-A1- 2015 245 958

## Description

### FIELD OF INVENTION

The present invention relates generally to absorbent garments and, particularly, absorbent garments having multi-layer folded thin absorbent cores. In more detail, the present invention relates to an absorbent core according to claim 1.

### BACKGROUND

Examples of disposable absorbent articles that are wearable by a user include baby diapers, training pants, and adult incontinence briefs and underwear, all of which may be made in disposable forms such as, for example, utilizing nonwoven materials. The terms "absorbent article" and "absorbent garment" refer to garments or articles that absorb and contain exudates and, more specifically, refer to garments or articles that are placed against or in proximity to the body of the wearer to absorb and contain the various exudates discharged from the body. These garments or articles, include diapers, training pants, feminine hygiene products, bibs, wound dressing, bed pads, and adult incontinence products. "Nonwoven" fabrics, according to an INDA definition, are broadly defined as sheet or web structures bonded together by entangling fiber or filaments (and by perforating films) mechanically, thermally, or chemically. They are flat, porous sheets that are made directly from separate fibers or from molten plastic or plastic film. They are not made by weaving or knitting and do not require converting the fibers to yarn. The basis weight of nonwoven fabrics is usually expressed as gsm or grams per square meter. In this context, "disposable" refers to articles which are designed to be discarded after a limited use rather than being laundered or otherwise restored for reuse. Disposable absorbent products have met with widespread acceptance in the marketplace for a variety of applications, including infant and adult incontinence care, in view of the manner in which such products can provide effective and convenient liquid absorption and retention while maintaining the comfort of the wearer.

Such disposable absorbent articles often include a topsheet that is configured to be closest to the wearer during use, a liquid-impermeable backsheet or outer cover, and an absorbent core between the topsheet and the backsheet. "Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. In some instances, such disposable absorbent articles also include an acquisition-distribution layer ("ADL") disposed between the topsheet and the absorbent core. "Absorbent core" means a structure positioned between a topsheet and backsheet of an absorbent article for absorbing and containing liquid received by the absorbent article and may comprise one or more substrates, absorbent polymer material, adhesives or other materials to bind absorbent materials in the core and, for purposes of the present invention, includes the disclosed absorbent laminate.

Over time absorbent cores used in such articles have become increasingly thinner with superabsorbent materials being included in ever-increasing amounts in place of traditional cellulosic pulp and other fillers and absorbents. While these thinner, superabsorbent-containing cores provide advantages, such as, generally offering a better fit to the wearer, they also present various challenges. One such challenge relates to the acquisition and distribution of liquid insults. In conventional core designs the liquid spreads radially from the point where it strikes, or insults, the core. Thus, rather than being dispersed across the core surface generally, its transport may be localized. This challenge is exacerbated by the issue of "gel blocking," which refers to the blocking of liquid transport through the core by the swelling and gelling of the superabsorbent material as it absorbs and retains liquid. Gel blocking may lead to leakage from the article when the core does not have the ability to absorb and retain liquid at a rate that meets or exceeds the rate at which the liquid reaches the core.

Prior art designs have attempted, to varying degrees of success and in a variety of ways, to address these issues. These efforts have involved the selection of superabsorbent materials based on the materials' properties, the addition of acquisition and distribution layers on top of the cores, and the positioning of the superabsorbent materials in the core in a variety of designs and arrangements.

Examples of certain absorbent cores and articles that address some or all of the foregoing issues are disclosed in U.S. Patent No. US 9,789,012 B2 (the '012 Patent). This '012 Patent discloses examples of absorbent laminates and folded multi-layer absorbent cores that include superabsorbent polymer particles ("SAP") and one or more layers of material such as, for example, tissue. "Layer" when used in the singular can be a single element or a plurality of elements. For example, a plurality of sheets may together define a single layer, such as, for example, a layer with a particular function to which the sheets of the layer contribute. "Lamination" is the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are a permanently assembled by heat, pressure, welding, or adhesives. "Superabsorbent" or "superabsorbent material" or "SAP" refers to a water-swellable, water-insoluble organic or inorganic material capable, under the most favorable conditions, of absorbing at least about 15 times its weight in an aqueous solution containing 0.9 weight percent sodium chloride and, more desirably, at least about 30 times its weight in an aqueous solution containing 0.9 weight percent sodium chloride and, even more desirably, at least about 50 times its weight in an aqueous solution containing 0.9 weight percent sodium chloride. The SAP materials can be natural, synthetic and modified natural polymers and materials. In addition, the SAP materials can be inorganic materials, such as silica gels, or organic compounds such as cross linked polymers.

Examples of certain laminates that can be used in absorbent articles can be found in PCT Application Publication No. WO 2018/112229 A1 (the '229 PCT Publication).

US2015/245958 A presents multi-layer, folded absorbent cores that are formed from an absorbent laminate comprising an absorbent layer between two tissue layers, in which the absorbent core includes a central channel running longitudinally along the core and crenellations profiled along the thickness of the core and providing enhanced liquid distribution across the core surface area or profile and improved liquid absorption into the laminate.

WO99/49826A1 discloses a disposable absorbent garment having a topsheet, a back-sheet and an absorbent core disposed between the topsheet and backsheet. The absorbent core is formed from a folded laminate comprising three layers, including an upper layer, a lower layer and a central fibrous layer. The central fibrous layer includes from about 50 percent to about 95 percent by weight super-absorbent polymer, and about 5 percent to about 50 percent by weight stabilization additives.

US2004/024375 presents an absorbent multi-layer material and a core comprising a central absorbent layer positioned between an upper layer and a lower layer, wherein at least of the upper and/or lower layer(s) have/has a vertical wicking index of greater than about 6 cm after 19 minutes and/or greater than about 3 cm after 1 minute.

### SUMMARY

The invention is defined by the independent claim 1. Advantageous embodiments are subjects of the dependent claims. This disclosure may include embodiments of multi-layer folded absorbent cores and absorbent articles and garments include such multi-layer folded absorbent cores that are not claimed, but may help to obtain a better understanding of the invention it use or the prior art. In sofar as the term *invention* or *embodiment* is used in the following, or features are presented as being optional, this should be interpreted in such a way that the only protection sought for is the claimed invention . References to "embodiments" through the description which are not under the scope of the appended claims merely represent possible exemplary executions that may help to better understand the claimed invention.

The present absorbent cores comprise an absorbent laminate. The absorbent laminate comprises: a first laminate layer comprising a tissue or nonwoven; a second laminate layer comprising a spunlace nonwoven; and an absorbent layer positioned between the first and second laminate layers, the absorbent layer comprising adhesive and greater than about 90 percent by weight super absorbent polymer (SAP). At least one of the first and second laminate layers comprises a through-air dried (TAD) tissue. Lateral portions of the absorbent laminate are folded inward toward a central longitudinal axis of the absorbent laminate such that multiple layers of absorbent laminate define a longitudinally folded absorbent core.

The second absorbent layer defines an outermost surface of the longitudinally folded absorbent core or the absorbent layer is a first absorbent layer, and the absorbent laminate further comprises: a third laminate layer comprising a spunlace nonwoven and disposed on an opposite side of the second laminate layer relative to the first laminate layer, wherein the third laminate layer defines an outermost surface of the longitudinally folded absorbent core; and a second absorbent layer disposed between the second and third laminate layers, the second absorbent layer comprising adhesive and greater than about 90 percent by weight super absorbent polymer.

In some of the foregoing configurations of the present absorbent cores, the longitudinally folded absorbent core defines a longitudinal channel. In some configurations, the channel has a width of from 10 mm to 30 mm.

In some of the foregoing configurations of the present absorbent cores, the first laminate layer comprises tissue.

In some of the foregoing configurations of the present absorbent cores, the absorbent layer(s) each comprises from 40 grams per square meter (gsm) to 80 gsm of the SAP. In some configurations, the total SAP content of all layers of the longitudinally folded absorbent core is from 200 gsm to 600 gsm, and/or a basis weight of the second laminate layer is greater than a basis weight of the third laminate layer.

In some of the foregoing configurations of the present absorbent cores, the longitudinally folded absorbent core has three or more layers of the absorbent laminate.

In some of the foregoing configurations of the present absorbent cores, the absorbent laminate has been mechanically softened by calendaring or temporary corrugating.

In some of the foregoing configurations of the present absorbent cores, the folding of the lateral portions of the laminate define folded lateral edges of the absorbent core, and the absorbent core defines a plurality of slits through at least one layer of the laminate, the slits extending from the lateral edges toward the central longitudinal axis.

In some of the foregoing configurations of the present absorbent cores, the longitudinally folded absorbent core has a plurality of sheets of the absorbent laminate.

In some of the foregoing configurations of the present absorbent cores, the absorbent core comprises a surge core and a base core, and at least one of the surge and base cores is defined by the longitudinally folded absorbent laminate.

In some of the foregoing configurations of the present absorbent cores, the less than 3 percent of the weight of the SAP comes from particles that will not pass through a 500 µm screen.

Some configurations of the present disposable absorbent articles comprise: a body-facing topsheet; a backsheet; and one or more of the present absorbent cores. Some of the foregoing configurations of the present disposable absorbent articles further comprise: an acquisition distribution layer (ADL) positioned between the topsheet and the absorbent core; where a width of the ADL at least 80% of a width of the longitudinally folded absorbent core.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The terms "substantially" and "about" are defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the term "substantially" or "about" may be substituted with "within [a percentage] of" what is specified, where the percentage includes 0.1, 1, 5, and 10 percent.

The terms "comprise" and any form thereof such as "comprises" and "comprising," "have" and any form thereof such as "has" and "having," and "include" and any form thereof such as "includes" and "including" are open-ended linking verbs. As a result, an apparatus that "comprises," "has," or "includes" one or more elements possesses those one or more elements, but is not limited to possessing only those elements. Likewise, a method that "comprises," "has," or "includes" one or more steps possesses those one or more steps, but is not limited to possessing only those one or more steps.

Any embodiment of any of the apparatuses, systems, and methods can consist of or consist essentially of - rather than comprise/include/have - any of the described steps, elements, and/or features.

Further, a device or system that is configured in a certain way is configured in at least that way, but it can also be configured in other ways than those specifically described.

The feature or features of one embodiment may be applied to other embodiments, even though not described or illustrated, unless expressly prohibited by this disclosure or the nature of the embodiments.

Some details associated with the embodiments described above and others are described below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. Views in the figures are drawn to scale, unless otherwise noted, meaning the sizes of the depicted elements are accurate relative to each other for at least the embodiment in the view.
FIGS. 1A-1C are a schematic views of embodiments of the absorbent laminates.
FIG. 2 is a schematic view of a first configuration of multi-layer folded absorbent laminates.
FIG. 3 is an enlarged view of one-half of the multi-layer folded core of FIG. 2.
FIG. 4 is a schematic view of a 3-layer configuration of folded multi-layer absorbent laminates.
FIGS. 5A and 5B are schematic views of 4-layer configurations of the folded multi-layer absorbent laminates.
FIGS. 6A and 6B are schematic views of 5-layer configurations of the folded multi-layer absorbent laminates.
FIG. 7 is a schematic view of a 6-layer configuration of folded multi-layer absorbent laminates.
FIGS. 8A-8D illustrate schematically folding schemes for respectively forming 3-layer, 4-layer, 5-layer and 6-layer folded multi-layer absorbent laminate cores.
FIG. 9 is a schematic view of one example of a particular 5-layer folded multi-layer absorbent laminate.
FIG. 10 shows dimensions of a particular example of the present 4-layer multi-layer absorbent laminate cores after each of two folds.
FIG. 11 schematically illustrates perspective and end views of a folded core with a terraced central channel defined separate layers of laminate partially surrounding an optional acquisition material in the interior of the core.
FIG. 12 schematically illustrates perspective and end views of an additional embodiment of a folded core with a terraced central channel defined separate layers of laminate partially, similar to that of FIG. 8 but omitting the interior acquisition material.
FIG. 13 is a schematic cross-sectional view of an absorbent article comprising two of the present folded multi-layer laminate cores.
FIGS. 14A-14B are schematic views of two embodiments of absorbent articles comprising two-part cores.
FIG. 15A depicts a table of total SAP basis weight for certain laminates in various configurations of the present folded multi-layer absorbent cores.
FIG. 15B depicts a table of certain performance characteristics for various laminate configurations, various folded multi-layer core fold configurations, and different types of SAP.
FIGS. 16A-16B depict perspective views of protective underwear conceptually illustrating buckling of a conventional fluff/SAP core and one of the present folded multilayer laminate cores, respectively.
FIG. 17 depicts a schematic view of a friction tester used to measure certain characteristics of the present laminates, which characteristics correlate to perceived smoothness.
FIG. 18 depicts a chart of coefficients of friction of certain of the present laminates, as derived using the friction tester of FIG. 16.
FIG. 19 depicts a schematic view of a device with a pair of corrugated rollers used to tenderize or improve perceived softness of certain of the present laminates.
FIG. 20 depicts a perspective view of one of the present folded multilayer absorbent cores with slits in its edges to improved perceived softness and flexibility of the core.

### DETAILED DESCRIPTION OF ILLUSTRATIVE EMBODIMENTS

The '012 Patent and the '229 PCT Publication are to be reviewed prior to studying the following:.

The present invention is directed to laminates and folded multi-layer absorbent laminates, in more detail to cores, that provide advantages over those in the prior art. For example, such advantages can include improved liquid acquisition, increased flexibility, and softer tactile feel perceived by a user handling an absorbent article containing or otherwise including one or more of the present folded multi-layer absorbent laminates.

### A. Absorbent Laminates

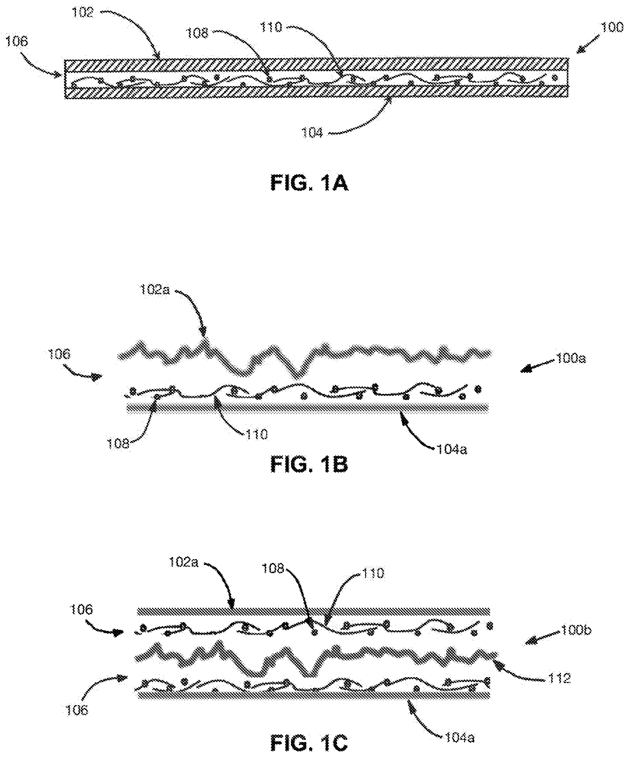

FIG. 1A depicts a schematic, cross-sectional illustration of a first embodiment 100 the present absorbent laminates. The depicted laminate 100 is configured for use in the present folded multi-layer absorbent laminates and cores, examples of which are described in more detail below. In the depicted configuration, laminate 100 comprises an upper laminate layer 102, a lower laminate layer 104, and an intermediate absorbent layer 106 between the upper and lower laminate layers.

Each of upper laminate layer 102 and lower laminate layer 104 may comprise or be constructed of a variety of materials, such as, for example, tissue or nonwoven. Examples of nonwovens include spunbond or carded webs of polypropylene, polyethylene, nylon, polyester and blends of these materials. In some embodiments, one or both of upper laminate layer 102 and lower laminate layer 104 comprises tissue. The tissue, for example, can be a porous tissue, a creped tissue, a standard tissue, or through-air dried ("TAD") tissue. One example of a tissue suitable for at least some of the present embodiments is the TAD variety such as TAD 4014282 tissue available from Dunn Paper in East Hartford, Connecticut, U.S.A. Another example of a tissue suitable for at least some of the present embodiments is a wet creped variety such as 3995 tissue, also available from Dunn Paper. Another example of a tissue suitable for at least some of the present embodiments is a high-creped variety such as 1113 tissue, also available from Dunn Paper. Another example of a tissue suitable for at least some of the present embodiments is 3995 tissue, also from Dunn Paper.

It is possible to print, or otherwise attach, SAP particles to a single layer of tissue or nonwoven and it is envisioned that these types of materials could also be used to make folded multi-layer absorbent cores as described below. Various adhesive and non-adhesive bonding methods are also known for laminating tissue and nonwovens. For example, mechanical bonds or stitching can be used to make bond multi-layer tissue laminates. By way of further example, synthetic fiber nonwovens can be bonded via known thermal or ultrasonic bonding techniques.

In some embodiments, one or both of upper laminate layer 102 or lower laminate layer 104 can comprise or be treated with a wet strength additive, such as, for example, Kymene^{™} from Solenis International, L.P. in Wilmington, Delaware. Such a wet strength additive can be applied, for example in "lanes," to the upper and/or lower laminate layers in the cross direction to strengthen the edges and/or control leakage at the side of a folded core. In other embodiments, one or both of the upper and lower laminate layers may comprise a skin-wellness ingredient and/or an odor-control ingredient.

Intermediate layer 106 includes particles of superabsorbent material 108 and an adhesive composition 110. The superabsorbent material can comprise a variety of materials, including organic compounds, such as cross-linked polymers. "Cross-linked" is a commonly understood term and refers to any approach for effectively rendering normally water-soluble materials substantially water insoluble, but swellable. Such polymers can include, for example, carboxymethylcellulose, alkali metal salts of polyacrylic acids, polyacrylamides, polyvinyl ethers, hydroxypropyl cellulose, polyvinyl morpholinone, polymers and copolymers of vinyl sulfonic acid, polyacrylates, polyacrylamides, polyvinyl pyridine and the like. Other suitable polymers include hydrolyzed acrylonitrile grafted starch, acrylic acid grafted starch, and isobutylene maleic anhydride copolymers, and mixtures thereof. Organic high-absorbency materials can include natural materials, such as agar, pectin, guar gum and peat moss. In addition to organic materials, superabsorbent materials may also include inorganic materials, such as absorbent clays and silica gels. Suitable examples of SAP include T9030, T9600, T9900, and Saviva polymers from BASF Corporation in Charlotte, North Carolina; and W211, W112A, W125, S125D, QX-W1482, QX-W1486, QX-W1504, and QX-W1505 from Nippon Shokubai Co. Ltd, N.A.I.I. in Houston, Texas; and AQUA KEEP SA50 II, SA55SX II, SA60N II, SA65S, HP500, HP500E (high-permeability), HP600, HP600E, HP650, and HP700E (high-capacity) from Sumitomo Seika Chemicals Co., Ltd. in Osaka, Japan.

The superabsorbent material typically is in particle form and can be of any desired configuration, such as granulated powders, fibers, agglomerated spheres and other shapes known to those skilled in the art. The particle size of the superabsorbent material may vary, but typically ranges from about 20 microns to about 1000 microns. Superabsorbent polymer particles, however, can impart roughness. This perceived roughness can be reduced in several ways. For example, the size of the SAP particles may be reduced. In particular, perceived softness may be improved when less than three percent (3%), for example less than two percent (2%), of the mass of the SAP in intermediate layer 106 is provided by particles that cannot pass through a 500 µm screen. Examples of superabsorbent polymers with this type of particle size distribution are SA50II, SA60NII, HP600, HP700NII, and HP700E from Sumitomo.

In addition to SAP selection or modification, surface roughness or perceived stiffness may be reduced by mechanical or structural means, such as by adding additional tissue or nonwoven or similar material between the laminate and the wearer. Alternatively, the first laminate layer (e.g., 102, 102a) and/or the second laminate layer (e.g., 104, 104a) of the present absorbent laminates can comprise a layer of through-air-dried ("TAD") tissue, through-air-bonded ("TAB") nonwoven, or spunlace nonwoven. For example, in the embodiment shown in FIG. 1A, each of upper laminate layer 102 and/or lower laminate layer 104 can comprise TAD tissue having a basis weight of from 10 gsm to 80 gsm, from 20 gsm to 70 gsm, from 30 gsm to 60 gsm, from 40 gsm to 50 gsm, from 10 gsm to 20 gsm, from 10 gsm to 30 gsm, or from 15 gsm to 25 gsm. By way of further example, each of upper layer 102 and/or lower layer 104 can comprise spunlace nonwoven having a basis weight of from 10 gsm to 80 gsm, from 20 gsm to 70 gsm, from 30 gsm to 60 gsm, from 40 gsm to 50 gsm, from 45 gsm to 55 gsm, from 10 gsm to 20 gsm, from 10 gsm to 30 gsm, or from 15 gsm to 25 gsm. In some configurations of the present folded multi-layer cores utilizing a three-layer laminate such as is shown in FIG. 1A or 1B, such as are depicted in the figures and described in more detail below, the upper or first laminate layer (e.g., 102, 102a) can form a majority of an outermost surface of the core and the bottom or second laminate layer (e.g., 104, 104a) can face inward for at least the outermost layer of the laminate; in other configurations, the bottom or second laminate layer (e.g., 104, 104a) can form a majority of the an outermost surface of the core.

Additionally or alternatively, softness can be improved by including an acquisition-distribution layers ("ADL") in an absorbent article that includes a fluffless core. Such an ADL may be disposed on the surface of or otherwise above the fluffless core to mask surface roughness of the core, as well as to improve acquisition and rewet performance. For example, such an ADL can comprise a through-air-bonded ("TAB") nonwoven with a basis weight of either of, or between, about 30 gsm and about 120 gsm. By way of further example, such an ADL can comprise one or more cellulosic fiber layers similarly disposed with a basis weight of either of, or between, about 100 gsm and about 350 gsm. Further, one or more TAB nonwovens and one or more cellulosic fibers layers can also be used in combination.

Additionally, the SAP particles in intermediate layer 106 may be uniformly or non-uniformly distributed within the intermediate layer. For example, in some embodiments, the SAP particles are distributed either uniformly or non-uniformly in the intermediate layer at a basis weight of either of, or between, 25 gsm and 70 gsm, such as, at a basis weight between 30 gsm and 40 gsm, or between 55 and 70 gsm. In the embodiment shown in FIG. 1A, intermediate layer 106 comprises SAP particles that are applied or otherwise distributed substantially uniformly at a relatively low basis weight, thereby forming substantially a single layer of SAP particles. However, a non-uniform SAP distribution in the absorbent laminate may be preferred in some embodiments to enhance z-direction liquid permeability through the laminate.

SAP distribution may be reflected by the measured Coefficient of Variation (COV) of the distribution. COV is defined as the standard deviation of basis weight of absorbent laminate samples divided by the mean basis weight and can be measured according to the following test. A circular die of 30 mm diameter is used to cut a total of 27 samples from an absorbent laminate according to the present invention. For a 500 mm wide x 385 mm length absorbent laminate used to make a folded, multi-layer core, as will be described herein in more detail in subsequent sections, a 3 x 3 array of samples is cut, in triplicate from three separate pieces of laminate. Each sample is weighed to determine its basis weight, and the coefficient of variation (COV) of basis weight is calculated for the laminate. The COV of basis weight is defined as (Std Dev of BW) / (Mean BW) x 100%. In preferred embodiments of the present invention, the COV of basis weight for absorbent laminates should be greater than about 5% but less than 25%.

As indicated, the intermediate layer of the absorbent composite 106 can include an adhesive composition. The adhesive composition should be of a type that is suitable for use in the production of disposable hygiene articles. In some of the embodiments, the adhesive composition is a thermoplastic hot-melt adhesive composition. A thermoplastic hot-melt adhesive composition generally comprises one or more polymers that provide cohesive strength, a resin or similar material that provides adhesive strength, possibly waxes, plasticizers or other materials that modify viscosity, and other additives, such as antioxidants and stabilizers. In some embodiments, the adhesive composition is a pressure-sensitive thermoplastic adhesive composition such as, for example, a synthetic rubber-based pressure sensitive adhesive composition having a glass transition temperature greater than 25°C. In certain embodiments, the adhesive composition may be a Styrene-Butadiene-Styrene (SBS) or Styrene-Isoprene-Styrene (SIS) block copolymer hotmelt adhesive composition. In this regard, these preferred adhesive compositions are described in U.S. Patent Application No. 14/632,963, entitled "Novel Absorbent Laminate for Disposable Absorbent Articles," filed February 26, 2015, which is highly recommended to be reviewed for all purposes and in a manner consistent with this application and invention. It is typically desirable to keep the amount of the adhesive composition in the intermediate layer at the minimum amount necessary to provide a laminate with acceptable integrity to be unwound at high speed in a converting process used to make absorbent articles containing the laminate.

The superabsorbent material and adhesive composition may be present in the intermediate layer in a variety of amounts, with some embodiments including the superabsorbent material as the majority component in the layer. In some embodiments, the superabsorbent material comprises at least about 90% of the total weight of the intermediate layer, for example, at least 94%, at least 95%, at least 97%, at least 98%, or at least 99%, of the total weight of the intermediate layer.

In some embodiments the absorbent laminate may utilize discrete acquisition cell (DAC) technology. This technology, and the inventions related to it, are described in U.S. Patent Applications No. 14/212,754, entitled "Absorbent Structure with Discrete Acquisition Cells," filed on 14 March 2014, and No. 14/212,969, entitled "Absorbent Structure with Dryness Layer," filed 14 March 2014, which applications are highly recommended to be reviewed for all purposes and in a manner consistent with this application and invention. DACs address the paradox of requiring high free volume for instantaneous liquid absorption in a low-volume thin structure. DACs provide an instantaneous increase in free volume in thin cores to rapidly absorb and contain free liquid before any appreciable swelling of SAP can occur and partition liquid into SAP over time so as to regenerate free volume in the DACs to absorb subsequent doses of liquid. Discrete Acquisition Cells can be comprised of compressed cellulosic sponge, creped cellulosic paper, soy bean hulls, and other filler materials that provide free volume for rapid absorption of liquid in thin laminates. In other embodiments, filler such as wood pulp or cellulosic fluff, may be mixed with the adhesive and SAP.

In embodiments where the absorbent layer contains Discrete Acquisition Cells, the superabsorbent material comprises at least about 40% of the total weight of the intermediate layer. Furthermore, in embodiments where the absorbent layer contains continuous filament or staple fiber tow, or continuous filament or staple fiber yarn, the superabsorbent material comprises at least about 40% of the total weight of the intermediate layer. In this regard, the basis weight of the SAP in the intermediate layer may range from about 10 grams per square meter (gsm) to about 400 gsm, preferably from about 40 gsm to about 150 gsm.

In some embodiments, and as shown in FIG. 1A, the left edge and the right edge of laminate 100 are open and substantially uncovered, for example, upper laminate layer 102 is not bonded to lower laminate layer 104 along the left and right edges. In other embodiments, adhesive may extend along one or both of the left and right longitudinal edges of the laminate such that upper laminate layer 102 is adhered to lower laminate layer 104 along such edge(s). In other embodiments, upper laminate layer 102 and lower laminate layer 104 may be joined together (*i.e.,* adhered or bonded) such that both of the left and right edges are sealed together and absorbent layer 106 is partially or totally encapsulated; however, as noted below, such joining is generally not necessary since the laminate, when formed into the multi-layer folded absorbent core, open edges of the absorbent laminate are typically not exposed in a way that could lead to SAP leakage.

The present absorbent laminates may be manufactured via processes that are known to those skilled in the art of absorbent article manufacturing. In one example of such a process, a roll or sheet of laminate can be made by metering a free-falling curtain of SAP particles and mixing the curtain of SAP particles with hot-melt adhesive fibers. This hot-melt adhesive fiber curtain can be formed using conventional hot melt spray equipment, such as the UFD applicator head provided commercially by ITW Dynatec in Hendersonville, Tennessee. The resulting mixture is then directed onto a moving substrate such as lower laminate layer 104, and a second substrate such as upper laminate layer 102 is directed on top of the SAP-adhesive mixture to form a sandwich structure. The fibrous layer of thermoplastic adhesive may be in at least partial contact with one or more of the superabsorbent particles, lower laminate layer 104, and upper laminate layer 102. The fibrous layer of thermoplastic adhesive may form cavities in which superabsorbent particles may reside, improving the immobilization of the superabsorbent particles. The fibrous thermoplastic layer may bond to the superabsorbent particles, lower laminate layer 104, and/or upper composite layer 102. In some embodiments, the superabsorbent particles are essentially dispersed throughout thermoplastic adhesive fibers. The laminate may then be rolled up and/or cut into segments sized for use in an absorbent article. Methods and apparatuses for metering SAP and mixing the SAP with hot melt adhesive are available commercially and known to those of ordinary skill in the art.

The present absorbent laminates may also exhibit SAP asymmetry. "SAP symmetry" in this context is defined as the ratio of the weights, or basis weights, of each of the tissue layers with attached SAP that is obtained upon separation of the laminate. For example, SAP asymmetry is equal to a value of 1 when the SAP is symmetric or equally distributed between the two layers of tissue. In a situation in which the laminate has a total basis weight of 133 gsm, for example 17 gsm tissue + 97 gsm SAP + and 2 gsm adhesive+ 17 gsm tissue, the SAP asymmetry would be approximately 5 if the laminate were to separate into layers of 111 gsm and 22 gsm. SAP asymmetry in this context is measured by heating a laminate to about 50 degrees Celsius for about 10 minutes and then separating the laminate by peeling the tissue or layers apart and then weighing each side. Some embodiments are configured to exhibit SAP asymmetry greater than about three (3), greater than about four (4), or about equal to five (5). In such embodiments that are incorporated into an absorbent core, the "weak side" or side that exhibits a lower basis weight in the SAP asymmetry test may be disposed to face a surface of the core that is expected in use to be first contacted or insulted by liquid. By way of example, SAP asymmetry may be imparted by sequentially applying two mixtures of SAP and adhesive fibers in distinct layers, having different SAP and adhesive ratios, to one of the laminate layers, such as lower laminate layer 104.

FIG. 1B depicts a schematic, cross-sectional illustration of a second embodiment 100a of the present absorbent laminates. Laminate 100a is substantially similar in many respects to laminate 100, and the differences will therefore primarily be described here. In particular, laminate 100a primarily differs from laminate 100 in that laminate 100a is depicted with dissimilar upper and lower laminate layers 102a and 104a. Specifically, in the depicted embodiment, upper laminate layer 102a comprises a TAD tissue, and lower laminate layer 104a comprises a different, non-TAD type of tissue. In one specific example, upper laminate layer 102a can comprise a TAD tissue (e.g., with a basis weight of from 10 gsm to 40 gsm, from 15 gsm to 35 gsm, from 20 gsm to 35 gsm, from 25 gsm to 35 gsm, or substantially equal to 28 gsm), and lower laminate layer 104a can comprise a creped tissue, for example a wet-creped tissue (e.g., with a basis weight of from 10 gsm to 35 gsm, from 15 gsm to 25 gsm, from 15 gsm to 20 gsm, substantially equal to about 17 gsm).

Some TAD tissues are "sided" or have a first side that is physically smoother than the second side, and the second side is less-smooth or deviates from being planar more than the first side. In embodiments including such sided TAD tissues, the smoother side may face outward. For example, in the configuration of FIG. 1B, the smoother side of the TAD tissue defining upper laminate layer 102a may face away from lower laminate layer 104a.

FIG. 1C depicts a schematic, cross-sectional illustration of a third embodiment 100b of the present absorbent laminates. Laminate 100b is substantially similar in many respects to laminates 100 and 100a, and the differences will therefore primarily be described here. In particular, laminate 100b primarily differs from laminate 100a in that laminate 100b includes an additional, medial laminate layer 112. Specifically, in the depicted embodiment, medial laminate layer 112 is disposed between upper laminate layer 102a and lower laminate layer 104a with two intermediate layers 106. As shown, one intermediate layers 106 is disposed between upper laminate layer 102a and medial laminate layer 112, and the other intermediate layer 106 is disposed between medial laminate layer 112 and bottom laminate layer 104a.

In some examples, upper laminate layer 102a can comprise a creped tissue, for example a wet-creped tissue (e.g., with a basis weight of from 10 gsm to 35 gsm, from 15 gsm to 25 gsm, from 15 gsm to 20 gsm, or substantially equal to 17 gsm); medial laminate layer 112 can comprise a TAD tissue (e.g., with a basis weight of from 10 gsm to 40 gsm, from 15 gsm to 35 gsm, from 20 gsm to 35 gsm, from 25 gsm to 35 gsm, or substantially equal to 28 gsm); lower laminate layer 104a can comprise a creped tissue, for example a wet-creped tissue (e.g., with a basis weight of from 10 gsm to 35 gsm, from 15 gsm to 25 gsm, from 15 gsm to 20 gsm, or substantially equal to 17 gsm); and intermediate layers 106 each can comprise SAP in an amount from 25 gsm to 100 gsm, from 40 gsm to 80 gsm, from 40 gsm to 60 gsm, from 60 gsm to 65 gsm, from 70 gsm to 80 gsm, substantially equal to 50 gsm, substantially equal to 62.5 gsm, or substantially equal to 75 gsm. In one such specific example, upper laminate layer 102a comprises a wet-creped tissue with a basis weight of 17 gsm, lower laminate layer 104a comprises a wet-creped tissue with a basis weight of 17 gsm, and medial laminate layer comprises a TAD tissue with a basis weight of 28 gsm, and intermediate layers 106 each comprise 50 gsm of SAP.

In other examples, upper laminate layer 102a can comprise a nonwoven, for example a spunlace nonwoven (e.g., with a basis weight of from 20 gsm to 80 gsm, from 30 gsm to 70 gsm, from 40 gsm to 60 gsm, or substantially equal to 50 gsm), medial laminate layer 112 can comprise a nonwoven (e.g., with a basis weight of from 10 gsm to 40 gsm, from 15 gsm to 35 gsm, from 20 gsm to 35 gsm, from 25 gsm to 35 gsm, or substantially equal to 28 gsm), and lower laminate layer 104a can comprise a creped tissue, for example a wet-creped tissue (e.g., with a basis weight of from 10 gsm to 35 gsm, from 15 gsm to 25 gsm, from 15 gsm to 20 gsm, or substantially equal to 17 gsm). In some configurations, the spunlace nonwoven of medial laminate layer 112 can comprise multiple types of fiber, for example polyester fiber and viscose fiber (e.g., 50% polyester fiber and 50% viscose fiber), and/or the spunlace nonwoven of first laminate layer 102a can comprise a single type of fiber, for example viscose fiber (e.g., 100% viscose fiber). In other configurations, the spunlace nonwoven of first laminate layer 102a can be comprised of polyester, viscose, and/or polypropylene fibers. In one such specific example, upper laminate layer 102a comprises a spunlace nonwoven with 50% polyester fiber and 50% viscose and a basis weight of 50 gsm, lower laminate layer 104a comprises a wet-creped tissue with a basis weight of 17 gsm, and medial laminate layer comprises a spunlace nonwoven with 100% viscose fiber and a basis weight of 28 gsm.

In some configurations of the present folded multi-layer cores utilizing a five-layer laminate such as is shown in FIG. 1C, such as are depicted in the figures and described in more detail below, the upper or first laminate layer (e.g., 102a) can form a majority of an outermost surface of the core and the bottom or second laminate layer (e.g., 104a) can face inward for at least the outermost layer of the laminate; in other configurations, the bottom or second laminate layer (e.g., 104a) can form a majority of the an outermost surface of the core.

In each of laminates 100a and 100b, the inclusion of the one or more TAD tissue or spunlace nonwoven layers can improve softness and liquid acquisition. For example, the overall thickness of the TAD tissue or spunlace nonwoven typically reduces the degree to which a user will perceive the texture or relative hardness of the superabsorbent particles in the intermediate layer(s). By way of further example, a TAD tissue can typically absorb more fluid than a similar area of creped or smooth tissue, a spunlace nonwoven can typically pass or convey fluid at a higher rate than a similar area of creped or smooth tissue, and/or the overall thickness and surface texture of a TAD tissue or spunlace nonwoven will provide additional physical distribution of the superabsorbent particles, for example in the thickness or Z direction, such that liquid can more readily flow between and to respective superabsorbent particles for absorption.

### B. Folded Multi-Layer Absorbent Cores

The above-described absorbent laminates, such as laminates 10, 10a, and 10b, can be folded to form multi-layer absorbent cores that provide improved properties and performance.

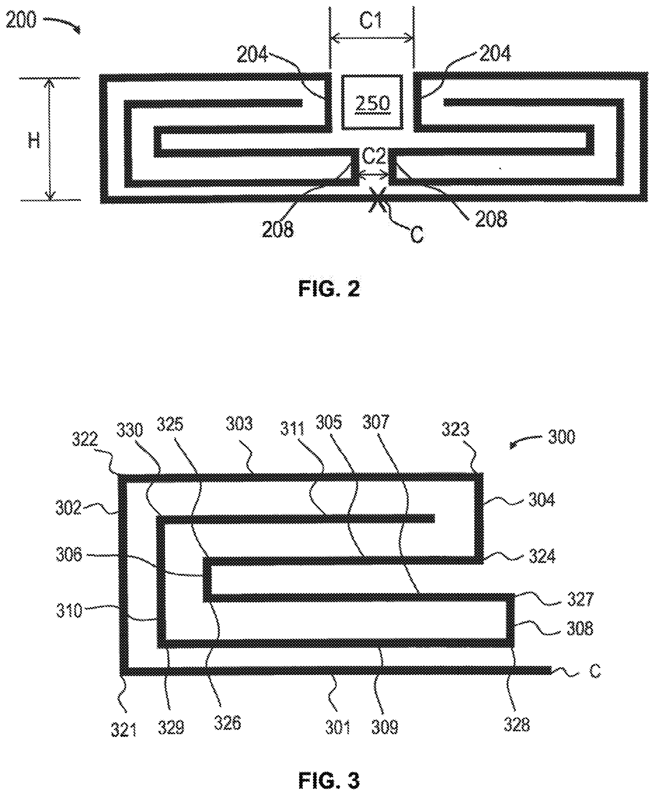

FIGS. 2-14B illustrate schematically various embodiments of the present folded multi-layer absorbent cores. These figures are exaggerated to better understand the overall structure of the cores and, as such, are for illustrative purposes only and are not necessarily to scale in the thickness or Z direction. Specifically, while these figures show individual laminate segments as generally horizontal and vertical, with the horizontal portions perpendicular to the vertical segments, such depictions are to illustrate the general folded configuration, the transitions from one segment of laminate to another, and the general relationship between the laminate segments, and are not intended to be limiting. More specifically, while the schematic views, and the following descriptions refer to "vertical" sections, it should be understood that, in application, the depth dimension, or "Z," is more compact and, thus, the "vertical" sections appear more as a transition area, or rounded folds, between generally horizontal laminate sections. Examples of typical values for the thickness of a single layer of the laminate and the 6-layer core are 0.4 - 0.5 mm and 2.4 - 3.4 mm, respectively, measured under a pressure of 2.5 g/cm². A typical value for the depth of the central channel in a 6-layer core is about 2.5 mm.

Such a multi-layer folded absorbent core configurations can increase the surface area of the absorbent laminate 100 that may be exposed to exudates and liquids *(i.e.,* the interfacial area) relative, for example, to single layer or non-folded cores. For example, certain embodiments of the present folded cores include internal surfaces that provide surface area of at least twice the base geometric surface area (*i.e.,* the footprint) of the folded absorbent core. In some embodiments, the interfacial area can be at least three times, at least four times, at least five times, at least six times, or more the base geometric surface area.

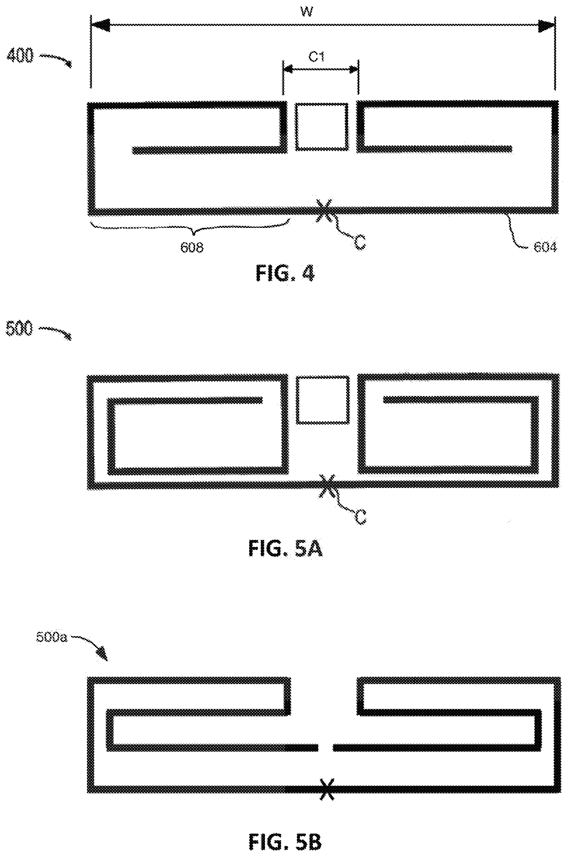

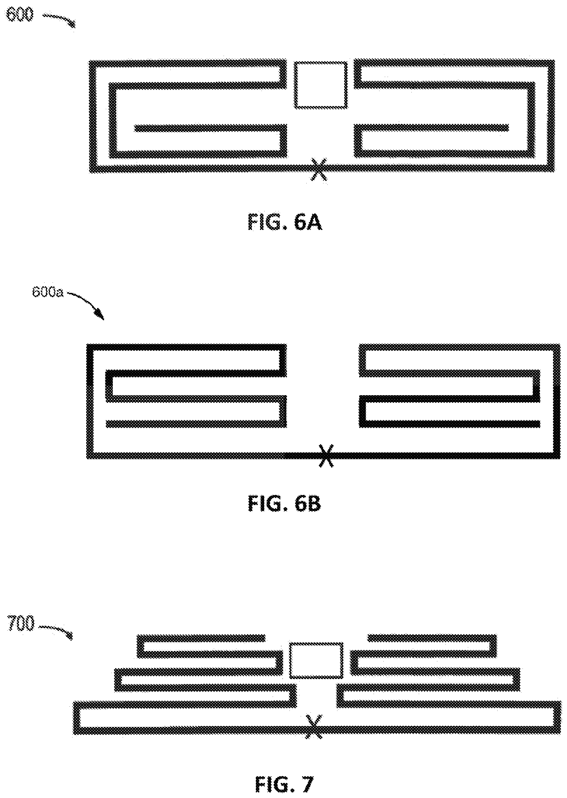

In addition to the increased surface area of the present folded multi-layer absorbent cores geometries, the folding of the laminate defines a plurality of additional internal liquid passageways, including crenellations formed by the folding of the absorbent laminate, that provide improved liquid acquisition and distribution performance. "Liquid passageway" refers to any means for liquid movement in the multi-layer core, including the internal crenellations. "Crenellation" refers to is internal indentation or crevice for liquid movement. By way of example, the 6-layer configuration of FIG. 2 provides two crenellations on each side of the central channel in direct communication with the central channel: a first crenellation between vertical segment 208 and the lowermost layer, and a second crenellation between vertical segment 204 and vertical segment 208. Likewise, the 5-layer configuration of FIG. 6 also includes two crenellations on each side of the central channel in direct communication with the central channel. By way of further example, in the "Christmas tree" or zig-zag fold design of FIG. 7, the absorbent laminate is folded into 6-layers but includes three crenellations on each side of the central channel in direct communication with the central channel. In contrast, the 3-layer configuration of FIG. 4 and the 5-layer configuration of FIG. 6 each defines a single crenellation on each side of the central channel in direct communication with the central channel.

In some embodiments, a sprayable adhesive, a wet-strength resin, and/or other material can be applied to the absorbent laminate to impart or improve wet strength of the tissue. For example, such materials can be applied selectively to only certain portions of the laminate, such as the outer peripheral portions of a folded core after the laminate has been folded.

Some of the present embodiments also define a central channel that can further improve liquid acquisition and distribution performance of the present cores. Such a central channel can, for example, provide a mechanism for receiving and temporarily containing large volumes of liquid (surges) and directing the bulk flow of liquid both longitudinally along the core and laterally within the core. As a result, core utilization is improved over that of a conventional fluff/SAP core that spreads liquid via a radial wicking mechanism. Furthermore, liquid travel in the present cores is enhanced by the multiple liquid passageways presented.

The internal crevices or interfaces are an important element of such liquid movement. The internal crevices or interfaces further enhance core utilization by moving laterally and longitudinally liquid from the central channel along and between the layers to significantly increase introduction of liquid to the larger interfacial core area. Such a mechanism is not burdened by the slower rate of liquid diffusion through the absorbent laminate in the z (top-to-bottom)-direction. Another advantage of the present folded cores is the that the channels and spaces between the folds create spaces where exudates and liquids may be contained until they can be absorbed into the absorbent layer, for example, by superabsorbent polymer in the absorbent layer. Yet another advantage of the present folded cores is that, in general, there is less side leakage, measured in laboratory liquid acquisition/rewet tests, relative to conventional cores because less liquid moves laterally outward to escape at the upper surface of a folded, multi-layer core. In some of the present embodiments, the central channel(s) and crenellations provide an internal or interfacial surface (the laminate-to-laminate interfaces providing a path for liquid spreading) that is greater than two times the surface area of the unfolded laminate.

Additionally, the foregoing advantages are even more pronounced, to unexpected degree, when utilizing the present absorbent laminates having at least one internal layer defined by a through-air-dried (TAD) tissue or spunlace nonwoven. Such a TAD tissue or spunlace nonwoven layer, for example, can improve fluid acquisition and transport through and between layers of the laminate, while also giving the core and article containing the core a softer, more-pliable feel as perceived by a user of such an article.

FIG. 2 depicts a schematic end view of one such multi-layer folded absorbent core 200, which is a folded 6-layer absorbent core. In this embodiment, a laminate - such as laminate 100, 100a, or 100b - has been folded to form two halves that are symmetrical relative to longitudinal centerline C and that define two central channels C1 and C2 that run substantially the length of absorbent core 200 along centerline C. While channel C1 is shown with a width that is greater than that of channel C2, channels C1 and C2 can in other embodiments have similar widths. Typical values for the thickness of a single layer of the laminate and the 6-layer folded core are 0.45 mm and 2.9 mm, respectively, measured under a pressure of 2.5 g/cm2. A typical value for the depth of the central channel is about 2.5 mm.

In some embodiments of the present cores with central channels, the width of the channel(s) may vary along the core's thickness or caliper, H. For example, in the embodiment of FIG. 2, first channel C1 is defined between opposing second vertical segments 204, and second channel C2 is defined between opposing fourth vertical segments 208. As shown in FIG. 2, first channel C1 may be wider than second channel C2 (C1>C2), providing a central channel with a greater width at the surface of the absorbent core 200 than at its base; however, in other embodiments, first channel C1 may be the same width as second channel C2 (C1=C2).

In some embodiments, the width of second central channel C2 can be less than 10 mm, for example equal to either of or between 0 mm to 5 mm, to provide more absorbency in the center of the core, and the width of first central channel C1 can be greater than the width of second central channel C2. For example, in some such embodiments, the width of first central channel C1 can be twice as large as the width of second central channel C2 or larger, for example, 50% or more of the width of the folded core. As a further example, in some embodiments, the width of first channel C1 is between 1 mm and 10 mm, and the width of second channel is greater than 5 mm, greater than 8 mm, and/or greater than 10 mm.

In those of the present folded absorbent cores that include a channel, the channel can provide improved and advantageous liquid acquisition time and rewet performance. As known in the art, liquid acquisition time is the time for a section of an absorbent element to absorb a known volume of liquid, typically saline, and rewet is the amount of liquid returned to the surface of the absorbent onto an absorbent filter paper when the absorbent is compressed by an external load. For most of the present embodiments of folded absorbent cores including a channel, at least one portion of the channel should be wide enough so as not to close during swelling of the absorbent laminate. For example, in the embodiment depicted in FIG. 1, at least first central channel C1 is wide enough to avoid closure during swelling of the laminate. In some embodiments, the width of central channel is equal to either of or between 8 mm to 50 mm, for example between 15 mm and 20 mm. Channel widths in these ranges can help compensate for the occasional "ruck" or overlap between the laminate defining the sides of the channel due, for example, in part to pressure applied to the sides of the core by the wearer during use.

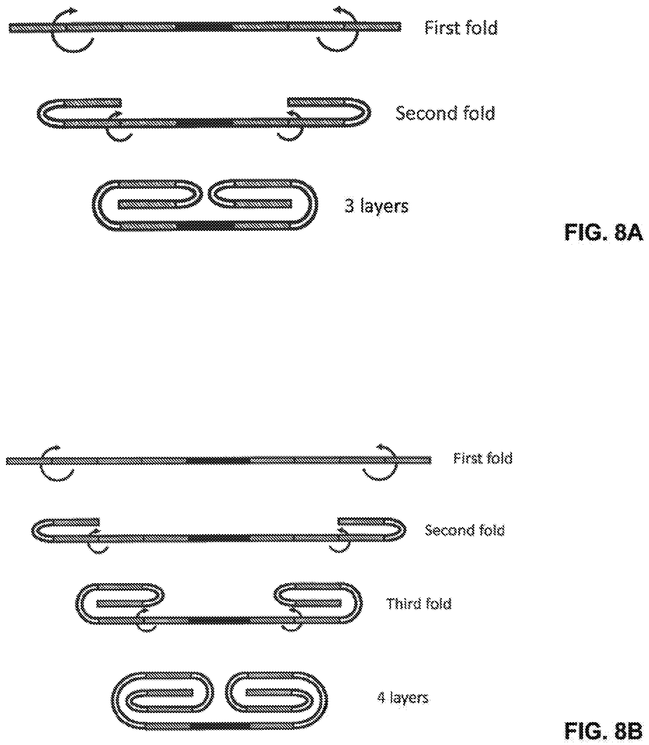

Swelling of the SAP in the laminate during liquid absorption may reduce the width of the channel and, as a result, may in some instances reduce performance. In some embodiments, it can therefore be desirable to provide at least a portion of the channel(s) with a sufficient width so as to avoid closure of the channel due to swelling of the SAP. In of the present some cores having a folded width of 80 mm to 120 mm, the channel has a width of between about 5% and about 45% of the folded core width and more preferably between about 8% and about 20%. Ultimately, the width of the channel(s) for a particular folded configuration will depend on the width of the unfolded laminate, the number of folds, and the overall width of the folded core. For example, in the 3-layer configuration of FIG. 8A, an absorbent laminate with a width of 533 mm can be formed into a 5-layer absorbent core of 115 mm width with a central channel width of 10 mm, in which example the width of the parent or unfolded laminate is 463% of the width of the folded core. According to another example, an absorbent laminate with a width of 533 mm can be formed into a 6-layer absorbent core of 100 mm width with a central channel width of 9 mm or a 6-layer core of 115 mm width with a central channel width of 15 mm.

As also shown in FIG. 2, some embodiments of the present folded multi-layer cores may comprise an optional insert 250, for example in the or a portion of the channel(s), such as to improve liquid acquisition performance and reduce end leakage. Additionally, after liquid absorption slows after the first few doses or insults, such an insert 250 can impede bulk liquid flow along the central channel to reduce or eliminate leakage from the front or rear of the core through the central channel. In other embodiments in which the channel includes different widths or two channels of different widths along the thickness or caliper, H, of the core, as shown in FIG. 2, the core may include two or more inserts, for example one insert in first central channel C1 and a second insert in second central channel C2. In other embodiments and configurations, the insert is omitted; as such, while shown in FIGs. 4, 5A, 6A, and 7, it should be understood that the insert may be included or omitted from these and other embodiments and configurations of the present absorbent cores and articles.

In some embodiments, the inclusion of an insert or inserts in the central channel may make it unnecessary or less important, relative to conventional fluff/SAP or prior fluffles cores, to include a conventional Acquisition Distribution Layer, or ADL, on the surface of the core. In some embodiments, insert 250 can include an acquisition material which may, for example, comprise cellulosic acquisition fiber and/or a nonwoven. Acquisition material 250 can, for example, be formed as pad of such fibers and/or wrapped or encased in a core wrap of tissue or nonwoven material.

In some such embodiments, insert 250 may comprise a cellulosic acquisition fiber. The cellulosic acquisition fiber may further comprise SAP. In preferred embodiments, the cellulosic acquisition fiber comprises no more than about 10% by weight SAP. A layer of cellulosic acquisition fiber could also be placed on the surface of a multi-layer folded core. A conventional (fiber or film) ADL is usually required on the surface of cellulosic acquisition fiber to improved overall dryness of the core.

In certain embodiments, channel insert 250 is about the same width as the channel into which it is inserted, for example, a first channel C1 as shown in FIG. 2 or a single channel of the core as shown in FIGS. 4-6. For example, insert 250 may have a width that is between 80% and 99%, for example, between 85% and 95%, of the channel or portion of the channel in which it is disposed. When the width of insert 250 is smaller than the width of the channel or portion of the channel in which it is disposed, additional central channels for liquid transport can be defined in gaps between the sides of pad 250 and vertical sections of laminate 204.

Additionally, in certain embodiments, channel insert 250 is at least about half the depth of the channel into which it is inserted. For example, insert 250 occupies substantially all of the depth of first channel C1, whereas the insert shown in FIG. 4 occupies substantially half of the channel in which it is disposed, and the insert shown in FIGS. 5-6. By way of further example, in other embodiments with two channels of different widths, such as first channel C1 and second channel C2 of FIG. 2, a single insert may span portions or all of the depths of both or all such channels. In some embodiments of the present cores that includes one or more inserts, one or more inserts each has portions disposed in the central channel and portions extending into or otherwise disposed in internal crenellations of the core such that the insert is wider than the channel or portion of the channel in which it is disposed, but not as wide the folded core.

In some embodiments, insert 250 can comprise an ADL-like nonwoven material that exhibits advantageous properties of acquiring the liquid insult and releasing and distributing the liquid across a broader area. For example, the insert(s) can comprise a through-air bonded ("TAB") ADL material, for example, comprised of bicomponent fibers treated with a durable or nondurable hydrophilic surface finish. By way of further example, the insert(s) can comprise melt-blown polypropylene or a low-twist yarn. In the case of low-twist yarns, the yarn may be comprised of polyester continuous filament or staple fibers with a durable, or alternatively non-durable, hydrophilic finish and, specifically, may range from about 1000 decitex to about 1500 decitex. In yet another example, the insert(s) can comprise a continuous filament or staple fiber tow or narrow-slit nonwoven carded or spunbond pulled from end of a spool to make a twisted, ribbon-like structure. In some embodiments, the insert(s) comprise a 60 gsm TAB ADL nonwoven material with a width of between about 5 mm and 15 mm. In alternative embodiments, the insert(s) can comprise discrete acquisition cell (DAC) technology. As noted above, Discrete Acquisition Cells can comprise compressed cellulosic sponge, creped cellulosic paper, soy bean bulls, and other filler materials that provide free volume for rapid absorption of liquid in thin laminates. Such DACs can be incorporated into the crenellations of a folded core or introduced into the core in an absorbent laminate.

While FIG. 2 depicts first channel C1 and second channel C2 opening upward, for example toward a topsheet of an absorbent article containing the core, in other embodiments, the folded multi-layer absorbent core can be inverted such that the core is orientated with the channels facing downward toward a backsheet of the article. In such other embodiments, liquid insults must pass through a single layer of absorbent laminate covering the central channel, which single layer can have sufficient porosity for the central channel and absorbent core to provide rapid liquid acquisition and spreading.

FIG. 3 is a schematic illustration of one-half of 6-layer absorbent core 200 shown in FIG. 2 comprising several horizontal and vertical segments and forming a folded core. By way of illustration only, each half 300 comprises a first horizontal segment 301 adjacent to the longitudinal centerline C, a first fold 321, a first vertical segment 302 adjacent to first horizontal segment 301, a second fold 322, a second horizontal segment 303 adjacent to first vertical segment 302, a third fold 323, a second vertical segment 304 adjacent to second horizontal segment 303, a fourth fold 324, a third horizontal segment 305 adjacent to second vertical segment 304, a fifth fold 325, a third vertical segment 306 adjacent to third horizontal segment 305, a sixth fold 326, a fourth horizontal segment 307 adjacent to third vertical segment 306, a seventh fold 327, a fourth vertical segment 308 adjacent to fourth horizontal segment 307, an eighth fold 328, a fifth horizontal segment 309 adjacent to fourth vertical segment 308, a ninth fold 329, a fifth vertical segment 310 adjacent to fifth horizontal segment 309, a tenth fold 330, and a sixth horizontal segment 311 adjacent to the fifth vertical segment 310.

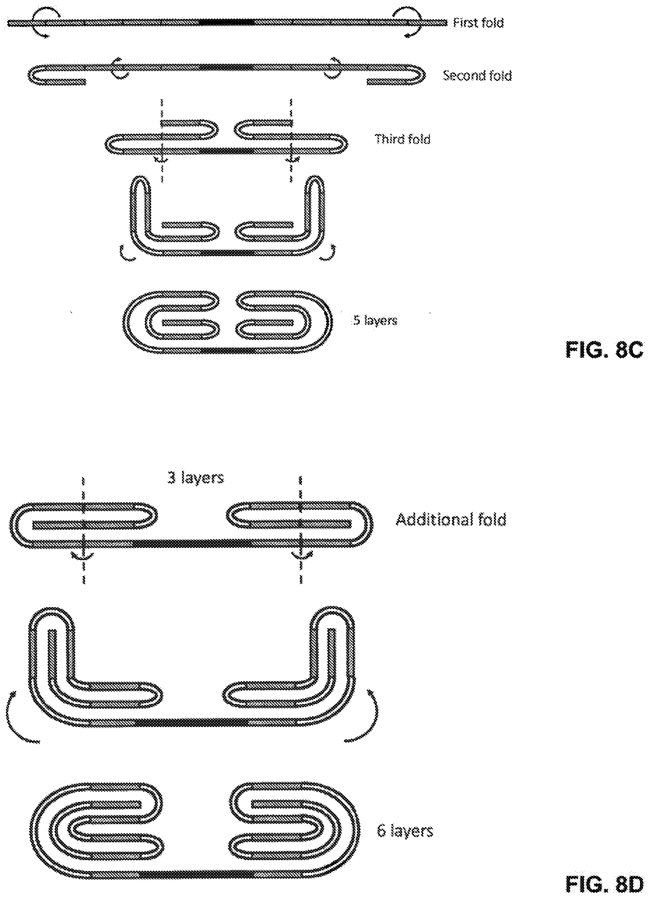

After folding, the six horizontal segments 301, 303, 305, 307, 309, and 311 form six layers of the folded absorbent laminate. In some embodiments, the lengths of the vertical segments are small compared to the lengths of the horizontal segments. Additionally, the vertical segments generally are in the form of fold, curves or transition areas from one generally horizontal segment to another, and not truly vertical segments, as indicated by the curved segments shown in FIG. 8D.

Other embodiments of an absorbent laminate may be similarly folded to form a folded multi-layer absorbent core with three, four, or five layers as shown, for example, in FIGS. 4 (three layers of laminate), 5A and 5B (four layers of laminated), and 6A and 6B (five layers of laminate), respectively. For example, laminate 100 may be folded to form an absorbent core 400 with three such layers (FIG. 4); an absorbent core 500 or 500a with four such layers (FIGS. 5A and 5B); or an absorbent core 600 or 600a with five such layers (FIGS. 6A and 6B).

For example, FIG. 4 depicts an absorbent core 400 in which a single sheet 604 of laminate is folded to define a core with an overall width W, a central channel having a width C1, and multi-layered lateral portions 608 each having a width of (W - C1) ÷ 2. As shown, each of multi-layered lateral portions 608 includes three layers of the laminate, such that the unfolded width of the sheet 604 of laminate is approximately W + (4 x (W - C1) ÷ 2) or W + 2 x (W - C1).

FIG. 7 schematically illustrates another embodiment of the present folded multi-layer folded absorbent cores. This core design provides enhanced liquid transport through the core by providing pathways or crenellations (internal indentations as will be described in following paragraphs) on the out-facing side of the core, in addition to the pathways from the central channel into the laminate. As can be seen, FIG. 7 schematically illustrates a "terraced" structure at both the outer edges and inner edges of each half of the multi-layer folded core. In alternative embodiments, either of the inner or outer edges may have a uniform edge profile while the opposing edge profile is terraced, or both inner and outer edge profiles are uniform.

The multi-layer folded absorbent cores described above may be made on standard converting machinery of the type typically used in the manufacture of disposable absorbent articles and the folds themselves may be made using a folding shoe. Embodiments comprising multiple pieces of laminate may also be manufactured using this same technique. An example of a typical folding shoe or board used in the industry is described in U.S. Patent No. 3,401,927, which is highly recommended to be reviewed for all purposes and in a manner consistent with this application and invention.

FIGS. 8A-8D schematically illustrate, by way of example, the steps performed when making 3-layer, 4-layer, 5-layer and 6-layer absorbent cores according to the present invention. To create a 3-layer structure, an absorbent laminate is folded two times. An initial 180 degree fold is made towards the center line at an axis at each end of the laminate (FIG. 8A). Then, a second 180 degree fold is made at each end in the same direction as the first fold, at axes closer to the centerline (FIG. 8A, second stage). The resultant structure comprises three layers (FIG. 8A, third stage).

To create a 4-layer structure, an absorbent laminate is folded three times. An initial 180 degree fold is made towards the center line at an axis at each end of the laminate (FIG. 8B, first stage). Then, a second 180 degree fold is made at each end in the same direction as the first fold at axes closer to the centerline (FIG. 8B, second stage). A third 180 degree fold is made at each end in the same direction as the first fold at another axis that is closer to centerline than each second axis fold (FIG. 8B, third stage). The resultant structure comprises four layers (FIG. 8B, fourth stage).

To create a 5-layer structure, an absorbent laminate is folded three times. An initial 180 degree fold is made towards the centerline at an axis at each end of the laminate (FIG. 8C, first stage). Note that the initial fold can be made in either an up-turned or downturned manner so as to provide different configurations for the final folded core. Then, a second 180 degree fold in the direction opposite to that of the initial fold is made at each end at an axis closer to the centerline of the laminate (FIG. 8C, second stage). The third 180 degree fold is made at the axes defined by the ends of the absorbent laminate, in the same direction as the first fold (FIG. 8C, third stage, dashed lines). The topology of the absorbent laminate structure during the third fold (third fold at 90 degrees) is demonstrated (FIG. 8C, fourth stage). The resultant structure comprises five layers (FIG. 8C, fifth stage).

To create a 6-layer structure, a folded 3- layer structure as described above is additionally folded once at each end. An additional 180 degree fold is made at the axis defined by the midpoint of the internal layer at each end, in the same direction as the folds used to create the 3-layer structure (FIG. 8D, first stage, dashed lines). The topology of the absorbent laminate structure during the additional fold (additional fold at 90 degrees) is demonstrated (FIG. 8D, second stage). The resultant structure comprises six layers (FIG. 8D, third stage).

The multi-layer folded absorbent cores provide significant flexibility to the selection and content of the SAP in the absorbent laminate and, in turn, in the multi-layer folded absorbent core. For example, as discussed above with respect to FIG. 1A, laminate 100 may have a SAP basis weight between about 40 gsm and about 150 gsm in some embodiments. Thus, the basis weight of SAP in a multi-layer folded absorbent core comprising six layers may range from about 240 gsm to about 600 gsm. In a preferred embodiment, the laminate has a SAP basis weight of about 60 gsm such that 6-layer absorbent core has a SAP basis weight of about 360 gsm. Alternatively, the multi-layer absorbent cores provides considerable design flexibility for adjusting core structure and an overall SAP basis weight. For example, to make a core with a total SAP basis weight of about 360 gsm, the absorbent laminate layer may have a SAP basis weight of about 120 gsm in a three-layer absorbent core, a SAP basis weight of about 90 gsm in a 4-layer absorbent core, and a SAP basis weight of about 72 gsm in a 5-layer absorbent core.

In certain embodiments, the vertical segments may bow or bend toward or away from centerline C. As mentioned previously, one of skill in the art would understand that a "fold" or "vertical" section is a location of transition between two generally horizontal segments and does not necessarily require a crease or other abrupt transition.

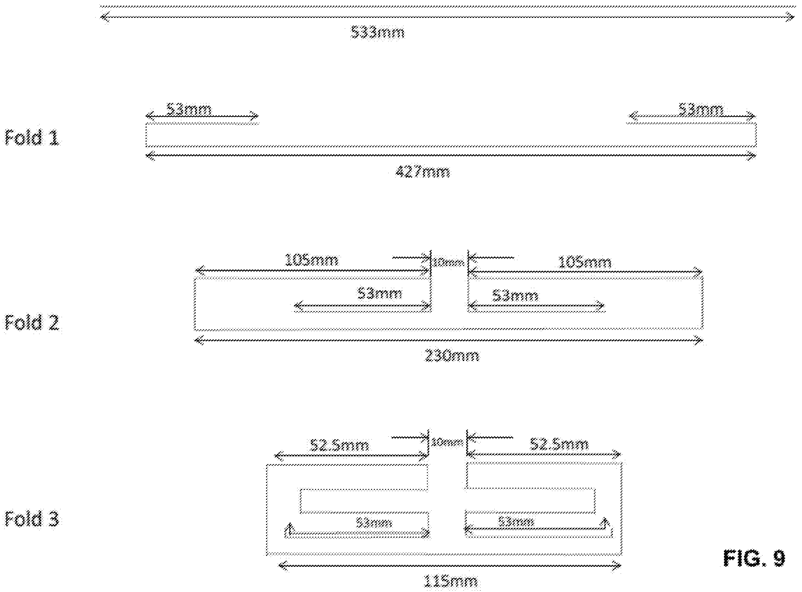

FIG. 9 is a schematic view of one example of a particular 5-layer folded multi-layer absorbent laminate, with dimensions shown in millimeters (mm).

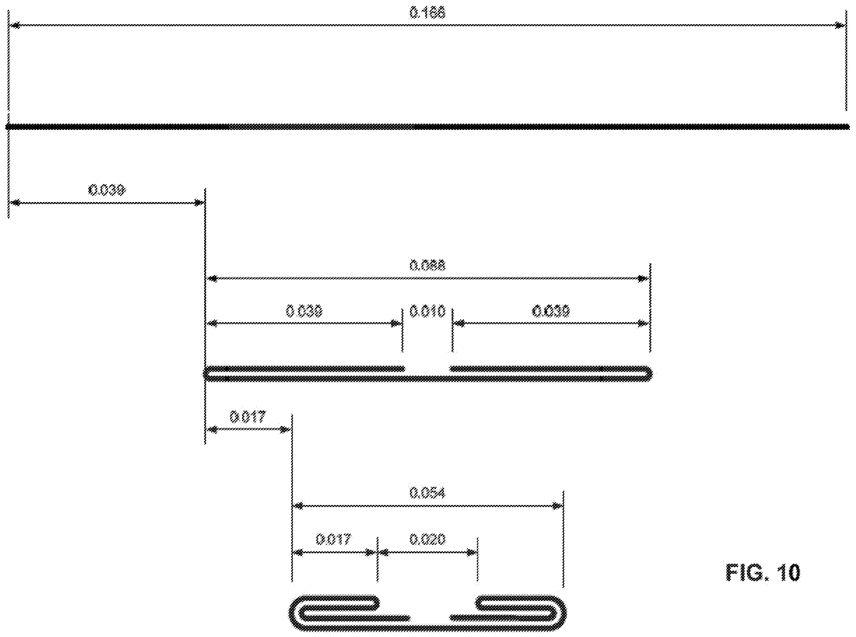

FIG. 10 shows dimensions of a particular example of the present 4-layer multi-layer absorbent laminate cores after each of two folds, with relative dimensions rather than absolute dimensions. For example, 0.166 is equal to 100% of the unfolded laminate width and the remaining dimensions are relative to 0.166, *i.e.,* 0.039 is a proportional part of 0.166. A prototype of the depicted embodiment was made for user in a size 4 diaper. In particular, a laminate with unfolded width of 533 millimeters was folded as indicated by the relative dimensions, *i.e.,* 0.166 = 533 mm, 0.039/0.166 = 125 mm, 0.088/0.166 = 283 mm, 0.010/0.166 = 32, and 0.017/0.166 = 55 mm, 0.020/0.166 = 64 mm. In the prototype, the core was positioned approximately 28 mm from the front of the diaper, and an acquisition-distribution layer with a width of approximately 108 mm and a length of approximately 149 mm was disposed about 50 mm from the leading edge of the core.

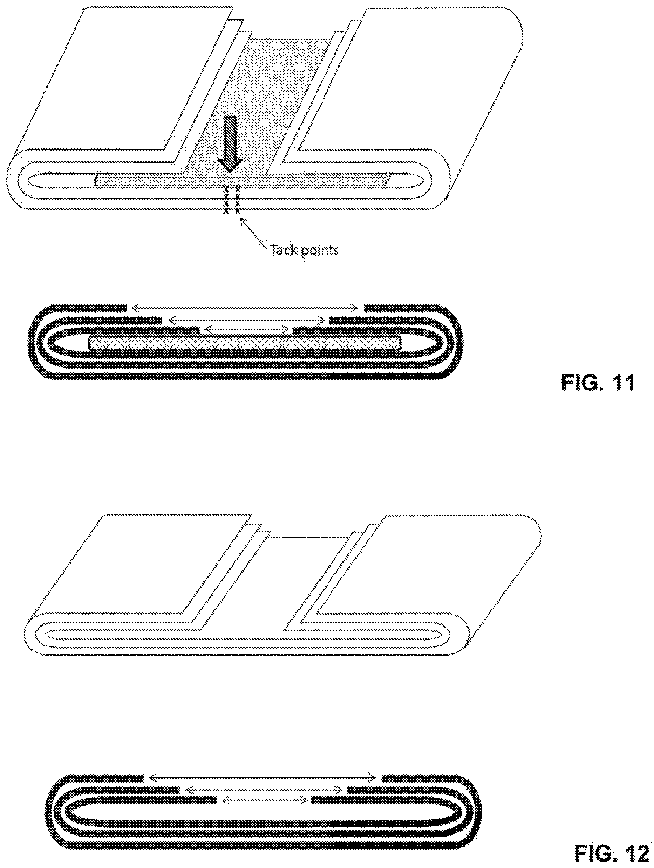

FIGS. 11 and 12 schematically illustrate additional embodiments of multi-layer folded absorbent cores in which a terraced or tapered central channel profile is formed by separate layers of laminate. This type of core is formed by stacking multiple pieces of absorbent laminate and then C-folding the outer sides of the laminates as shown such that the edges of the folded layers of laminate define a tapered or terraced central channel, as shown. When pieces of material with the identical unfolded widths are used, the difference in radii of the respective folds will cause the channel width to taper as shown. However, if a more-pronounced degree of taper or terracing is desired, pieces of absorbent laminate with different unfolded widths can be used. This embodiment is beneficial as the folding occurs in a single step and has the added benefit of suppressing the feel of the edges of the channel as the channel is only three layers thick and the edges taper outwardly. In addition, the wider opening of a tapered or terraced central channel helps liquid to flow into the interior of the core when that liquid impinges the surface of the core at a distance from the center of the open channel. The embodiment of FIG. 11 includes an optional layer of acquisition material in the center of the core which is wrapped by the separate layers of laminate. The acquisition material can be comprised, for example, of cellulosic or nonwoven acquisition fiber, or DAC materials of the type previously described.

### C. One-Part and Two-Part Folded Multi-Layer Cores

Some embodiments of the present absorbent articles include just one of the present folded multi-layer absorbent cores (a "One-Part" core), whereas others of the present absorbent articles include one of the present absorbent cores and an additional absorbent core (a "Two-Part" core). The additional absorbent member second core may be a single layer absorbent laminate, one or more other folded multi-layer absorbent cores, a conventional absorbent core with SAP/fluff or fluff only, or a combinations thereof. Such two-Part cores can provide zoned absorbency to increase absorbency in a part of the product where absorbency is needed.

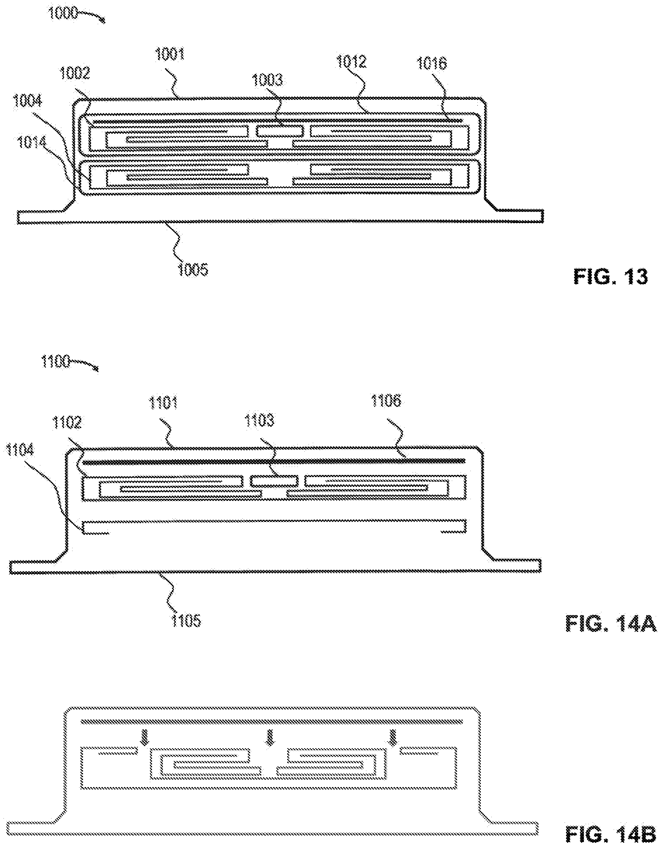

FIG. 13, FIG. 14A, and FIG. 14B schematically illustrate embodiments of absorbent articles that comprise Two-Part absorbent cores.

According to the embodiment shown in FIG. 13, an absorbent article 1000 comprises topsheet 1001, a first multi-layer folded core according to one embodiment of the invention, which will be referred to as a "surge" core 1002, optional channel insert 1003, a second multi-layer folded core according to the present invention, which will be referred to as a "base" core 1004, and a backsheet 1005. In the illustrated embodiment, surge core 1002 additionally comprises a layer of cellulosic acquisition fiber 1016 positioned above the multi-layer folded core itself to improve liquid acquisition performance. Cellulosic acquisition fiber has a higher Absorption Against Pressure ("AAP") value and a lower Centrifuge Retention Capacity ("CRC") value than that of fluff pulp. AAP and CRC are parameters well known to those skilled in the disposable absorbent article field. The AAP test method is described in EDANA WSP 242.3 (10), and the CRC test method is described in EDANA Test Method WSP 241.2.R3 (12) . AAP is a measure of an absorbent material's ability to absorb a 0.9% saline solution against a 0.7 psi load. CRC is a measure of the amount of 0.9 wt% saline solution that an absorbent material can retain after free swell and centrifugation to remove bulk interstitial liquid. The acquisition fiber layer 1016 absorbs liquid rapidly, temporarily holds it with capillary tension, and partitions the liquid over time to the core below. Cellulosic acquisition fiber is well known to those skilled in the art. In an alternative embodiment, a layer of cellulosic acquisition fiber can be placed into the central channel to improve liquid acquisition performance. This acquisition fiber, in both cases, can be used with or without SAP and, if SAP is included, levels of about 10% or less are preferred.

In an alternative embodiment, conventional (fiber- or film-based) ADL can be placed on the top surface of the folded surge core to provide additional dryness. Similar to the cellulosic acquisition fiber, the ADL can be folded within a multi-layer folded core to impede rapid spreading of high volumes of liquid in the central channel. Additionally, the ADL can assume a variety of widths and lengths depending on, among other parameters, the core width. In general, ADL widths approximately equal to the width of the multi-layer core, or at least about 95% of the core width, are preferred.

It also has been determined that placement of an ADL relative to the front edge of the absorbent core (known as "offset") affects overall leakage. In this regard, preferred performance has been discovered when the ADL is offset from the core's front edge. For example, cores according to the present invention exhibited reduced leakage results when the ADL is offset from the core's front edge, by at least about 25 mm and, in some cases, at least about 50 mm or greater.

Absorbent articles, particularly baby diapers, oftentimes include stand-up barrier cuffs that reduce side leakage in use. These cuffs are generally adhered or "tacked down" at their ends to the wearer-side article surface. It has been discovered in accordance with the present multi-layer core design that leakage results are affected by the position of this tack down relative to the core's front edge. Particularly, it has been discovered that improved leakage results are obtained with the novel core designs of the present invention when the barrier cuff is free-standing along the length of the core and is tacked down approximately at the front edge of the core, but not overlaying the core itself.

FIG. 13 also shows schematically that the absorbent cores 1002 and 1004 are enclosed and retained by a wrap material 1012 and 1014. Core wraps are well known in the art and may be constructed from, for example, tissue or nonwoven material. However, because of the excellent core stability of the multi-layer folded cores of the present invention, it will be possible, and in many cases preferable, to use a multi-layer core in an absorbent product without any additional tissue or nonwoven core wrap.

It has further been determined that the leakage performance of cores of the current invention can be improved by selection of a SAP that has an optimal liquid absorption time for the particular dimensions of the core. For example, SAPs with a 0.9% saline absorbency time in the range of about 160 seconds to about 220 seconds provide improved absorbency before leakage in a baby diaper than SAP's with absorption rates below about 160 seconds.

Additionally, in a preferred embodiment, zoned absorbency may be implemented in a Two-Part core to make efficient use of the absorbent materials. More specifically, in a Two-Part core, the surge layer may be shorter than the base core to provide more absorbent material and absorbency in the area of insult and less core and absorbency in areas of less insult and liquid. For example, some embodiments, the base core may be about 80 to about 120 mm wide and about 345 to about 400 mm long, while a surge core may be about 80 to about 120 mm wide and about 215 to about 260 mm long.

In another embodiments of a Two-Part core, shown in FIG. 14B, the partial length surge core is comprised of a folded, multi-layer core (in this example a 6-layer core) that has a folded width that is about 20 mm less than the width of the central channel formed by a folded, multi-layer core (in this example a 3-layer core) of the lower, full length base core. This core presents three central channels to a wearer of the absorbent article containing the core. This embodiment is particularly effective at acquiring liquid that might impinge the core to one side of the central channel formed by the surge core and run off to the side of the product, such as when the absorbent article is being used with the subject lying on their side.

In some instances, Two-Part cores can be made with different SAP's in each core. For example, a more permeable SAP may be included in the upper, or surge, core laminate for improved liquid acquisition and a higher capacity SAP may be included in the lower, or base, core laminate for higher liquid capacity. Alternatively Two-Part cores can be made with an upper, surge layer comprised of a multi-layer absorbent core containing a higher capacity SAP and a lower, base layer comprised of a lower capacity, slower absorbing, higher permeability SAP to improve spreading and core utilization. In some embodiments, it is advantageous to include acquisition materials and DACs in the laminates used to make the surge core.

FIG. 14A is a schematic cross-sectional view of another embodiment of an absorbent article 1100 having a Two-Part core. As shown, this core comprises topsheet 1101, surge core 1102, channel insert 1103, and backsheet 1105. According to this embodiment, the base core 1104 comprises a C-folded layer of absorbent laminate located below surge core 1102. The C-fold will seal the laminate edges to the backsheet under itself and eliminate migration of hydrated SAP from the free ends of the laminate. This, however, is usually not necessary as that SAP is well-constrained within the laminate. An ADL 1106 is located above surge core 1102 and channel insert 1103. In other embodiments, base core 1104 may be a single unfolded layer. In yet additional embodiments, base core 1104 may comprise a mixture of conventional fluff and SAP, or may comprise only conventional fluff.

Similar to Two-Part cores, a One-Part core would include the standard topsheet and backsheet, and optionally an ADL, as schematically illustrated in FIG. 13 and FIG. 14A for Two-Part cores, but would instead utilizing only one multi-layer absorbent core. Such one-part cores may be easier and/or less-expensive to manufacture on a converting machine.

In both One-Part and Two-Part absorbent cores, the geometry and dimensions of the core or cores may vary. For example, in embodiments configured for use in a baby diaper, a One-Part core may be between about 200 mm and about 450 mm long, for example between about 345 mm and about 385 mm long; between about 60 mm and about 120 mm wide, for example about 110 mm wide; and between about 2 mm and about 6 mm in thickness or caliper, for example about 3.1 mm in thickness or caliper. In some embodiments of a Two-Part core, the upper surge core is between about 215 mm to about 245 mm long, the folded width of the surge core is about 100 mm wide, the folded core is about 3.8 mm in thickness or caliper. In such embodiments, the lower base core can be between about 345 mm to about 385 mm long, and from about 100 mm to about 120 mm wide, and a thickness or caliper of about 4 mm. The lower base core of this preferred embodiment could be made from either a folded laminate or a single layer of unfolded laminate.

The present folded multi-layer absorbent cores are, in in most embodiments, greater than 2 mm in thickness or caliper. Such cores are, however, formed by folding a material that is much thinner. By way of example, a 1066 mm diameter roll of the laminate will yield at least 3100 lineal meters of material. Such a roll would yield over 8500 cores and, if it were running at a production rate of 400 products per minute, would run for longer than 21 minutes. Roll run time over 15 minutes is considered not unreasonable for those skilled in the art. This would not be possible if the core had to be unwound from a roll in its final thickness, *i.e.,* in a folded state, and the relatively lower roll run time for prior art core technologies that require that cores be unwound from their rolls in their final thickness therefore presents a serious problem for such prior art core technologies.

Core placement within the absorbent article can also be important. Some embodiments place the leading edge of the core within about 30 mm, and preferably less, of the front edge of the diaper chassis. Another relative measure regarding the placement of the core is its location relative to the frontal tape that is often part of an absorbent article's design. Preferably, the leading edge of the core is positioned slightly behind the frontal tape relative to the absorbent article's front edge.

Some embodiments for the Two-Part core design, include (a) a 6-layer surge core comprising 45-97 gsm S125D SAP per layer and having a length of 215 mm combined with a single-layer absorbent laminate comprising 89 gsm W211 SAP and having a length of 345 mm; (b) a 5-layer surge core comprising 45-97 gsm W125 SAP per layer and having a length of 215 mm combined with a single layer absorbent laminate comprising 97 gsm W125 SAP and having a length of 385 mm; and (c) a 5-layer surge core comprising 45-97 gsm SA55SX II SAP per layer and having a length of 215 mm combined with a folded, 2-layer absorbent laminate comprising 89 gsm SA55SX II SAP and having a length of 385 mm. In each case, both surge and lower cores have a folded width of about 110 mm and a central channel having a width in the range from about 10 to about 20 mm.

Some embodiments for the One-Part core design, include (a) a 6-layer core comprising about 45-97 gsm S125D SAP per layer, (b) a 5-layer core comprising 45-97 gsm W125 SAP per layer, and (c) a 5-layer core comprising 45-97 gsm SA55SX II SAP per layer. The core can have a length of from about 345 mm to about 385 mm, a width of about 110 mm, and a central channel width of from about 10 mm to about 20 mm.

The present folded multi-layer absorbent cores may be manufactured using conventional converting equipment. For example, large pancake rolls can be utilized to handle the absorbent laminate, thus avoiding the need for expensive separate processes for spooling or festooning. Similarly, the laminate can be folded in a relatively straightforward process, for example, by use of a folding shoe, or in other ways that will be well known to those skilled in the art. The process experiences little to no SAP loss during conversion because the SAP is confined between tissue or nonwoven layers. Additionally, the process offers ample opportunity to increase line speeds on an off-line laminate process to reduce raw material cost. Alternatively, it may be possible to reduce cost by making the laminate for the multi-layer core on-line.

The present folded multi-layer folded absorbent cores and the absorbent products that incorporate such cores present improved and unexpected results when compared with conventional cores. For example, the multi-layer cores exhibit improved liquid acquisition resulting from the central channel, crenellations, high internal surface area, and wicking between adjacent upper and lower layers. Additionally, the cores exhibit good core utilization with the central channel moving liquid in longitudinal and lateral directions and improved core stability and integrity in use.

The present folded multi-layer absorbent cores typically display high SAP efficiency due to the low SAP basis weight in the individual layers of laminate and allow the use of higher capacity SAPs with moderate permeability. More specifically, high absorbency against pressure (AAP) and high SAP efficiency in a multi-layer laminate can be obtained with superabsorbent polymers of higher centrifuge retention capacity (CRC) than can otherwise be used in thin cores without fluff pulp. For example, certain preferred SAPs may exhibit a CRC value of about 33-38 g/g. Similarly, certain preferred SAP's exhibit a Saline Flow Conductivity (SFC) value between about 0 and about 10 x 10⁻⁷ cm³ sec/g. Saline Flow Conductivity, another measure well-known in the disposable absorbent article field and described, for example, in U.S. Patent No. 5,599,335, measures the permeability of a swollen hydrogel layer.

The multi-layer structure of the present folded absorbent cores also typically improves performance of the SAP. The ability to successfully use superabsorbent polymers with relatively low AAP and high CRC in the absorbent cores of this invention contrasts with current pulpless core designs which have used superabsorbent polymers with relatively high AAP, low CRC and high permeability (*i.e.,* SFC > 20 x 10⁻⁷ cm³ sec/g). A superabsorbent polymer with high values of SFC and 0.7 AAP has a relatively low CRC capacity, and more of this type of SAP will be needed to provide the liquid capacity required of an absorbent core to function.

In addition, the present folded multi-layer absorbent cores have excellent liquid containment, exhibiting no side leakage in testing. The cores offer manufacturing advantages as well. Specifically, they can be produced with moderate run times and by use of simple folding equipment well known in the art. Also, the inventive cores experience manufacturing savings in that they do not require a nonwoven core wrap.

In still another advantage, the multi-layer absorbent cores exhibit decreased thickness or caliper when compared to conventional fluff/SAP cores. The present cores therefore have advantages for making more discreet, garment-like absorbent products that require less packaging and can be stored and shipped at lower cost.

### D. Performance Characteristics of Folded Multi-Layer Cores

The present folded multi-layer cores (MLCs) utilize multiple layers of thin (relative to conventional fluff/SAP cores) laminates that comprise one or more substrate layers and one or more absorbent layers of SAP and adhesive. Such laminates can be folded (e.g., as described above) to form an MLC with a central, longitudinal channel. Within such a channel, the folds and layers of the present MLCs define a large surface area of laminate to facilitate liquid acquisition and absorption while mitigating potential SAP gel blocking by distributing the SAP in distinct layers. The present MLCs generally have high permeability that is driven by the core structure itself rather than SAP properties alone, which can result in levels of SAP efficiency that are higher than what could be achieved in fluff/SAP cores with high SAP concentrations. As a result, using high-capacity SAPs in the present MLCs can meaningfully reduce SAP usage and associated SAP cost relative to conventional fluff/SAP cores. Additionally, the present MLCs generally provide improved liquid containment, especially for reducing side/leg leakage, and have significant tensile strength when wet, and provide improved core stability and integrity in use.

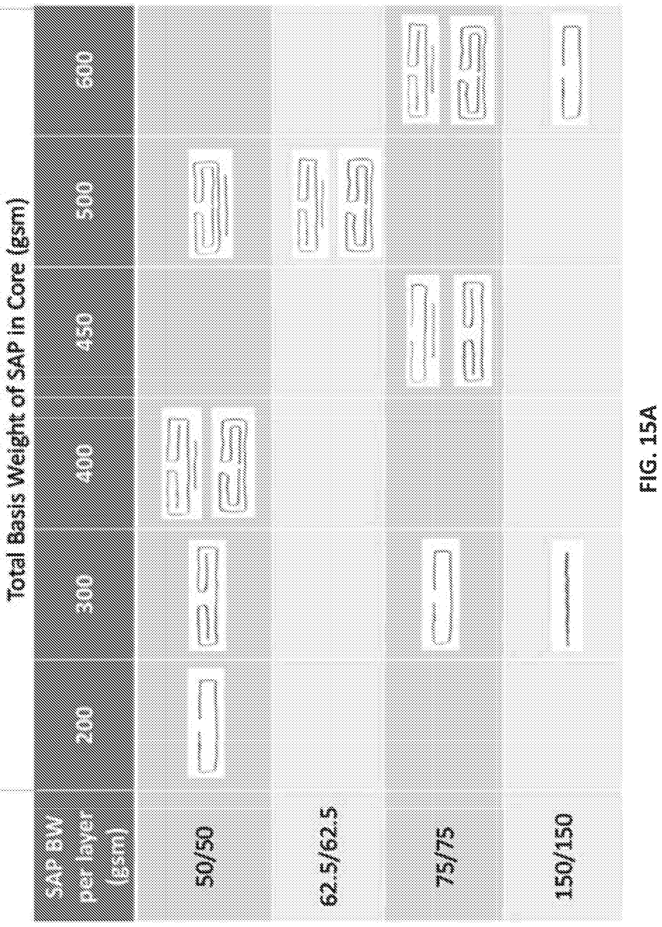

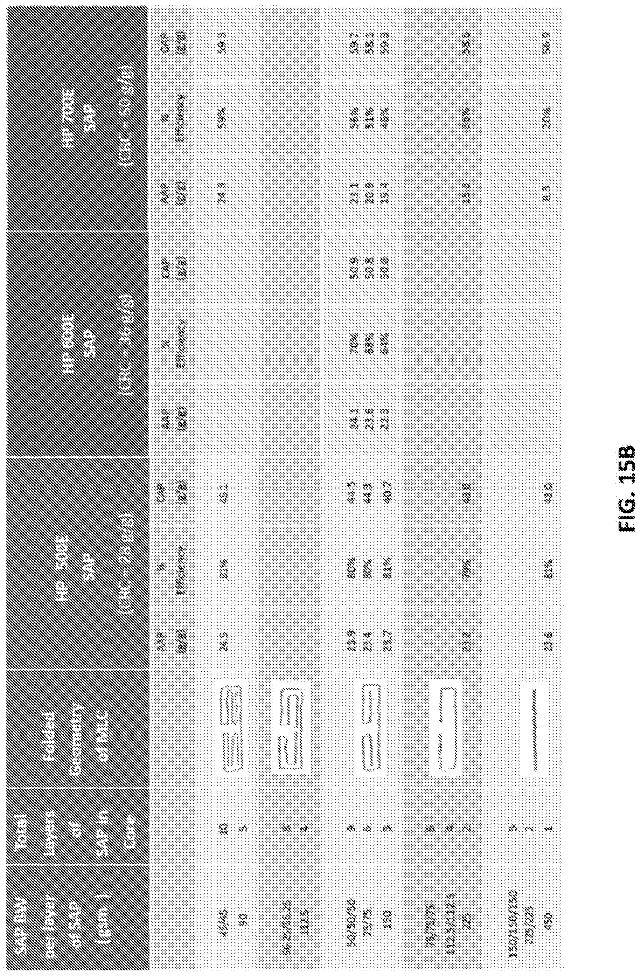

Certain variables can be adjusted to achieve a level of absorbency in an MLC desired for a particular application. For example, the number of layers of SAP and substrate in a laminate, the type and basis weight of SAP in each layer, the composition of the substrates (typically cellulosic tissue or synthetic fiber nonwoven), and the number of layers of laminate provided in a folded core can all impact the absorbency characteristics of an MLC. This is illustrated in the design matrix of FIG. 15 for absorbent cores with total basis weights of SAP in the range of 200 to 600 gsm, levels appropriate for heavy AI and baby diaper products. Total basis weights of SAP are typically lower for BCP's and liners for light incontinence, but general factors for laminate and core design remain similar.

FIG. 15A illustrates a number of configurations of MLCs that were produced and investigated to varying degrees. The laminates used in the folded MLC cores illustrated in the table of FIG. 15A were comprised of three layers of substrate and two layers of SAP, as illustrated in FIG. 1C. Specifically, first laminate layer 102a comprised a 50 gsm spunlace nonwoven with 50% polyester fiber and 50% viscose fiber; second laminate layer 104a comprised a 17 gsm wet-creped tissue; medial laminate layer 112 comprised a 28 gsm spunlace nonwoven with of 100% viscose fiber. Each intermediate layer 106 comprised SAP in an amount of from 50 to 150gsm (as indicated in the first column-e.g., 50/50, 62.5/62.5, 75/75, 150/150). The present laminates (and MLCs) can be constructed with either high-permeability SAP (e.g., Sumitomo HP500E) or high-capacity SAP (e.g., Sumitomo HP700E); in general, high-permeability SAP (e.g., HP500E) provides more compressional resiliency when hydrated, and may therefore provide improved user perception of softness and compliance in some applications.

### 1. SAP Efficiency

Improvements in SAP efficiency and the resulting ability to use higher-capacity SAPs in a thin core are some of the main reasons for the utility of MLC core designs. The specific absorbency for an absorbent core can be expressed as the Absorbency Against Pressure (AAP) at 0.7 psi of a 60 mm diameter circular section cut from a front portion of the core. Units of AAP can be expressed in grams of urine absorbed per cm² of core area (g/cm²) or grams of urine absorbed per gram of SAP (g/g). After an equilibrium value of AAP is obtained after 30 min., the pressure is removed and the free swell capacity (CAP) of the core section is determined. Finally the pressure is re-applied to determine Retention Under Load (RUL), with similar units. RUL is the maximum capacity that the core material can achieve under a pressure of 0.7 psi, therefore the ratio of AAP/RUL x 100% (with both AAP and RUL expressed in the same units) is a measure of the efficiency of the core (or laminate). The contributions of tissue and/or nonwoven in the core are determined experimentally and used to calculate the absorbency of the SAP alone. Absorbency of the SAP is expressed in units of gram of saline absorbed per gram of SAP (g/g). Results in the table of FIG. 15B show how SAP efficiency changed as a function of the basis weight of SAP in each layer, the number of layers of SAP in each laminate, and the number of layers of laminate in each core -- all for cores with a total SAP basis weight of 450 gsm. HP500E is a high-permeability SAP with a CRC of about 28 g/g (determined with the WSP Method), and HP700E is a high-capacity SAP with a CRC of about 50 g/g (determined with the WSP Method).

In FIG. 15B, the first column indicates the number of intermediate absorbent layers of SAP (and adhesive) and the basis weight of SAP in each intermediate absorbent layer. For example, "45/45" indicates a laminate with two intermediate absorbent layers (e.g., as shown in FIG. 1C) each with 45 gsm of SAP; and "90" indicates a laminate with one intermediate absorbent layer (e.g., as shown in FIG. 1A or 1B) having 90 gsm of SAP. Similarly, "50/50/50" indicates a laminate with three intermediate absorbent layers each with 50 gsm of SAP-i.e., with two medial laminate layers (e.g., 112) such that a first intermediate absorbent layer is disposed between a first outer laminate layer (e.g., 102a) and a first medial laminate layer, a second intermediate absorbent layer is disposed between the first medial laminate layer and a second medial laminate layer, and a third intermediate absorbent layer is disposed between the second medial laminate layer and a second outer laminate layer (e.g., 104a).

In general, SAP efficiency and softness/resiliency in hydrated cores improves with decreasing SAP basis weight in individual layers; however, lower SAP basis weights require more layers of substrate, which can lead to unsatisfactory increases in core stiffness, rigidity, and handle.

One example (the "PUW Core Configuration") of a core suited for protective underwear (PUW) or baby diapers was constructed with a first laminate layer of 50 gsm spunlace nonwoven with 50% polyester fiber and 50% viscose fiber, a second laminate layer of 17 gsm wet-creped tissue, an intermediate laminate layer of 28 gsm spunlace nonwoven with 100% viscose fiber, and two intermediate absorbent layers each having 75 gsm of HP500E SAP. This laminate was folded to provide an MLC with three layers of laminate (e.g., as in FIG. 4) and a central channel with a width of 20 mm, with the 50 gsm spunlace nonwoven laminate layer defining the outermost surface of the MLC. This MLC had a core length of 440 mm and an unfolded laminate width of 260 mm, providing 17.6 g of SAP per core. In contrast, a conventional fluff/SAP core for a commercial PUW product could contain about 15.5 g of fluff and 15.5 g of SAP.

SAP Efficiency of the high-permeability HP500E SAP was generally independent of the construction of the laminate and had a constant value of about 80%, suggesting that a SAP with a value of CRC even greater than 28 g/g could be used efficiently in these MLC cores. In cores made with laminates containing a higher-capacity (but lower permeability) HP600E SAP (i.e., with a CRC of about 36 g/g), SAP efficiency generally increased with decreasing SAP basis weight. However, it is worth noting that the 23.6 g/g AAP of the higher-capacity HP600E SAP in the 75/75 two-SAP layer core was comparable to or greater than that of the 23.4 g/g value obtained for the high-permeability HP500E SAP. However, in the laminate, the HP600E achieved a greater free swell capacity (CAP) than that of HP500E (i.e., 50.8 g/g vs. 44.3 g/g). A somewhat more-permeable SAP, like HP600E, can be expected to provide a good AAP (e.g., greater than ~20 g/g) and a higher CAP (e.g., greater than ~44 g/g) in this same 75/75 two-SAP layer core. The inventors were surprised to learn that a SAP like HP700E with an even-higher CRC of about 50 g/g could provide excellent performance in an MLC core. For example, the higher capacities offset the lower SAP efficiencies to provide surprisingly high values of AAP. In one particular example, the AAP of 20.9 g/g provided by HP700E SAP in the 75/75 two-SAP layer core was relatively high, even though not quite as high as the 23.6 g/g AAP provided by the HP600E SAP. The CAP of 58.1 g/g provided by the HP700E SAP, however, was higher than the CAP of 50.8 g/g provided by HP600E SAP. For comparison, HP700E SAP used in a conventional fluff/SAP core with about 50% SAP would have an AAP value of less than 10 g/g. Cost of an absorbent core can be reduced, while maintaining acceptable performance, by providing higher values of CAP while maintaining AAP at a target value of at least about 1.0-1.3 g/cm².

### 2. Buckling

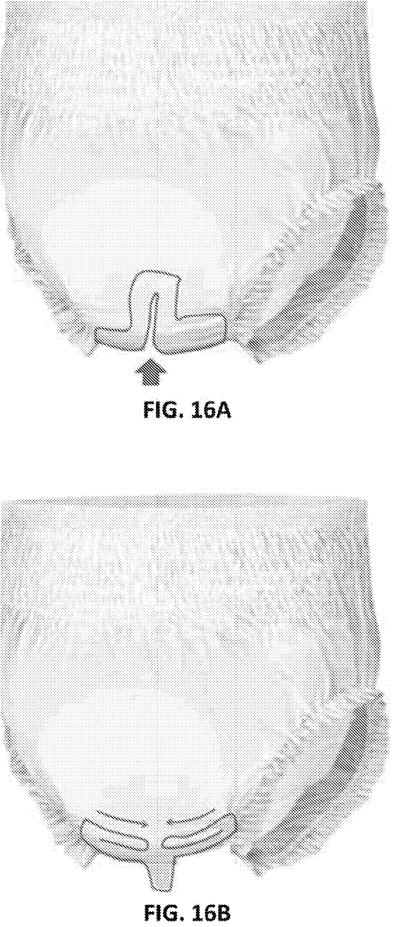

For an MLC width of about 100-120 mm, it can be beneficial to have a central channel width of about 20 mm (e.g., the PUW Core Configuration described above). This channel width allows the sides of the core to move laterally, and permits the floor of the core to buckle in a downward direction when compressed between the legs of the user, as shown in FIG. 16B. The result is a core configuration, in use, that provides a geometry that is both better suited for containment and capable of providing a significant improvement in comfort. In contrast, a conventional fluff/SAP core almost always buckles in an upward direction when compressed between the legs, as shown in FIG. 16A, especially when the core is partially hydrated. This upward buckling makes it easier for urine to run off of the core during use. The overall thickness of such typical conventional cores in both vertical and in-plane directions, and the resistance of such cores to lateral compression in those directions, can make for an uncomfortable wearing experience for a user.

US Patent Application Publication US 2017/0273835 (the '835 Publication) describes absorbent cores containing at least one channel for reducing Wet Compression Force of a hydrated core and Relative Wet Caliper Increase between the legs of a subject. The '835 Publication teaches a preference for a Wet Compression Force below 27 N and a Relative Wet Caliper Increase less than about 30%, possibly suggesting that its hydrated core compressed more easily certain directions with reduced increases in thickness. However, the '835 Publication does not appear to encourage or recognize the benefits of downward buckling.

### 3. Lab Performance

The PUW Core Configuration MLC and a conventional fluff/SAP core were further tested for various performance characteristics such as AAP, CAP, RUL, Acquisition (ACQ), and Rewet (REW). In particular, three MLC samples weighted from 3.27 g to 3.62 g were tested and compared to three conventional fluff/SAP samples weighted from 2.88 g to 3.00 g. In general, the PUW Core Configuration performed as well or better than the conventional fluff/SAP core, with the potential to be more cost-effective that the conventional fluff/SAP core.

**AAP:** The average AAP in g/cm² of the MLC samples exceeded that of the fluff/SAP samples, while the average AAP in g/g of the MLC samples was slightly less than that of the fluff/SAP samples. The improvement in AAP on a g/cm² basis suggested an improved per-area core performance relative to the conventional fluff/SAP core.

**CAP:** The average CAP in both g/cm² and g/g of the MLC samples exceeded that of the fluff/SAP samples, suggesting improved core performance relative to the conventional fluff/SAP core.

**RUL:** The average RUL in both g/cm² and g/g of the MLC samples exceeded that of the fluff/SAP samples, suggesting improved core performance relative to the conventional fluff/SAP core.

**ACQ/REW/Leak:** Conventional ACQ, REW, and Leakage performance for the PUW Core Configuration MLC were also better than for the conventional fluff/SAP core. These tests utilize multiple insults or doses of simulated urine. For the first dose, the acquisition time for the MLC was somewhat higher than that for the fluff/SAP core, but acquisition times were significantly lower (i.e. better) for subsequent doses. REWET and Leakage were also better for the MLC core.

**Absorbency Before Leakage (ABL):** In a Sitting Mannequin leakage test, performance of the PUW with the TIP MLC core was comparable to that of the commercial PUW product made with a fluff/SAP core. Each had an ABL value of about 450 g. (at 300 ml/min), which is adequate for the intended use of PUW products.

### E. Perceived Softness and Smoothness of Folded Multi-Layer Cores

The present laminates with five or more layers (and MLCs using such laminates) generally become softer as they become hydrated and, unlike conventional fluff/SAP cores, they do not go through a "hard transition" as they become saturated in use. Additionally, however, user perception of MLCs utilizing such laminates can be improved by enhancing the softness and smoothness of dry MLCs before they become hydrated, for example to improve comfort when worn. Such laminates and MLCs can be smoothed and softened in the dry state by one or more of various approaches: (1) using a finer particle size distribution of SAP; (2) improving basis weight uniformity of SAP in the laminate; (3) light calendaring to "flatten" adhesively bonded agglomerates of SAP; (4) using smoother substrates for the surface of the laminate next to skin; and/or (5) mechanical tenderization such a temporary corrugation.

### 1. SAP Particle Size Distribution

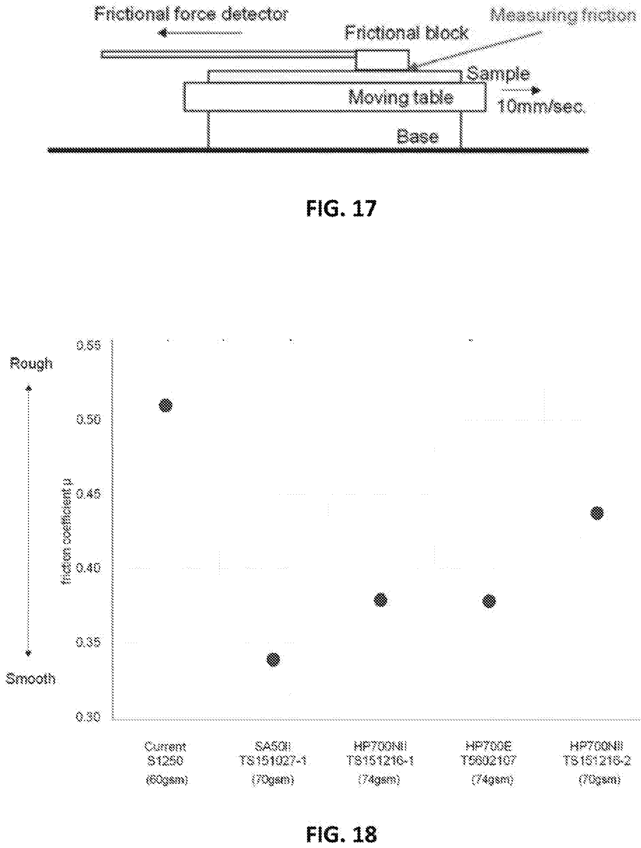

To investigate the impact of SAP particle size distribution on smoothness and perceived softness, different three-layer laminates were formed by distributing a single layer of SAP and adhesive between two layers of 17 gsm wet-creped tissue. The dry coefficient of friction was then measured for each of the laminate samples using a KES-SE-STP Friction Tester available from Kato Tech Co. Ltd. and schematically illustrated in FIG. 17. In particular, laminates were made with five different SAP-basis weight combinations with SAP content basis weights of 60 gsm to 74 gsm and 7% adhesive (by weight relative to basis weight of SAP). The SAPs were different in the mass fraction of the amount of polymer residing in particles that could pass through a 500 µm screen as shown in Table 1 below.

**TABLE 1**

| No. | Sample | Remark | Distribution of Particle size (%) | | | | | |
|---|---|---|---|---|---|---|---|---|
| | | | 850µm on | 500µm on | 250µm on | 180µm on | 106µm on | 106µm pass |
| A | Current | S1250 | 11.8 | | | | | |
| B | SA50II | 3410046 | 0.0 | 0.0 | 48.5 | 41.9 | 7.9 | 1.7 |
| C | HP700NII | TS151216-1 | 0.0 | 0.2 | 92.6 | 6.4 | 0.4 | 0.4 |
| D | HP700E | T5602107 | 0.0 | 1.4 | 82.9 | 12.8 | 0.8 | 2.1 |
| E | HP700NII | TS151216-2 | 0.0 | 2.4 | 89.2 | 7.0 | 0.6 | 0.8 |

The coefficient of friction is the frictional force divided by the normal force applied between the sliding surfaces. The dry coefficient of friction of laminates (equilibrated at 22°C and 50% relative humidity) of these laminates decreased as the mass fraction of particles over 500 µm decreased, as shown in Table 1 and FIG. 18. These differences in the coefficient of friction were meaningful as determined by subjective perception by consumers. Ultimately, smoothness and perceived softness was best when less than 5%, and preferably less than 2.5%, of a mass fraction of SAP particles were larger than 500 µm.

### 2. Calendaring

Certain of the present laminates comprising different SAPs were calendared and evaluated for smoothness with a Tissue Softness Analyzer (TSA) available from emtec Electronic GmbH. The TSA is a multifunctional measuring instrument used to assess the softness, smoothness/roughness, stiffness, and elasticity of tissue and fabrics. A combination parameter HF (Facial II algorithm), which represents the overall subjective haptic feeling of a material, was calculated by its proprietary software. HF has been shown to correlate with subjective perceptions of tissue softness. Higher values of HF indicate a softer feel. A second parameter, TS750, is a quantitative measure of the vibration of the sample during the test, such that the smaller the value, the smoother the surface.

In particular, laminates were constructed of three laminate substrate layers and two intermediate absorbent layers of SAP, as shown in FIG. 1C. Samples were made with either T9900 SAP (BASF) or HP500E SAP (Sumitomo). The upper and lower laminate layers each comprised a 17 gsm wet-creped tissue. The medial laminate layer comprised a 28 gsm spunlace nonwoven containing 100% viscose fiber. All of the laminate samples contained either two intermediate absorbent layers of SAP at 50 gsm per layer, or two intermediate absorbent layers of SAP at 75 gsm per layer, each with adhesive in an amount of about 7% of the mass of the SAP in each layer. Of the T9900 SAP (BASF), 19% of the mass resided in particles that would not pass through a 500 µm screen. The finer HP500E SAP (Sumitomo) had about 2% of the mass of the sample residing in particles greater than 500 µm. Single layers of laminate samples were calendared between steel rolls set at gaps of 0.75 mm (light calendaring) and 0.55 mm (moderate calendaring). In other work, this laminate was calendared on a winder between steel rolls at a nip pressure / load in the range of 10-40 lb /meter.

Before calendaring, the 50/50 gsm SAP laminate made with T9900 SAP had a caliper of 1.127 mm and the 75/75 gsm SAP laminate had a caliper of 1.209 mm. Laminates made with the finer HP500E SAP, had calipers of 0.951 mm and 1.063 mm, respectively, for laminates made with 50/50 and 75/75 gsm SAP. After calendaring, laminates made with the coarser T9900 SAP remained thicker than those made with HP500E SAP.

Calendaring improved perceived smoothness and softness for all of the samples. Values of TS750 (dB) decreased-i.e., the laminates became smoother-as the gap between the calendar rolls became smaller. Laminates made with the finer HP500E SAP were smoother than those made with the T9900 SAP. Laminates containing the finer HP500E SAP that were calendared at a 0.75 mm gap were highly preferred for smoothness and softness.

In a further iteration, a similar laminate was made with 75/75 gsm HP500E, but in which the upper laminate layer comprised (instead of tissue) a 50 gsm spunlace nonwoven with 50% polyester and 50% viscose fibers. This improved liquid acquisition, as well as provided for a smoother surface against the skin of the wearer. Values of TS750 measured on the side of the laminate with the 50 gsm spunlace nonwoven were much lower (i.e smoother) than those measured for the laminate with the outer strata of tissue-i.e., 52.74 dB (CD) and 54.85 dB (MD) for 50 gsm spunlace nonwoven versus 118.68 dB for tissue. Calendaring this laminate at a gap of 0.75 mm improved the softness even more, in both MD and CD directions (i.e., 39.62 dB for CD and 40.81 dB for MD), versus 90.15 dB for calendared tissue, thus providing a very soft and smooth absorbent core.

When calendaring such laminates, care must be taken to not damage the SAP. For example, AAP values are sensitive to SAP damage caused by crushing of individual SAP particles. AAP results obtained for laminates containing HP500E showed no evidence of SAP damage when they were calendared at the 0.75 mm gap.

Without being limited to a particular theory, calendaring is believed to impart smoothness to a laminate due to the disruption and flattening of aggregates containing many SAP particles. These aggregates are formed when depositing SAP and adhesive to form an intermediate absorbent layer, and tend to increase as the COV of SAP basis weight increases. The surface becomes smoother when the bumps formed by these aggregates are flattened or eliminated. Calendaring is a good way to smooth and soften laminates that had been made with high values of COV of SAP basis weight. Individual particles of HP500E SAP, all of which are less than 0.50 mm in dimension, do not suffer deformation or damage when calendared in a laminate at a gap of 0.75 mm.

### 3. Temporary Corrugating

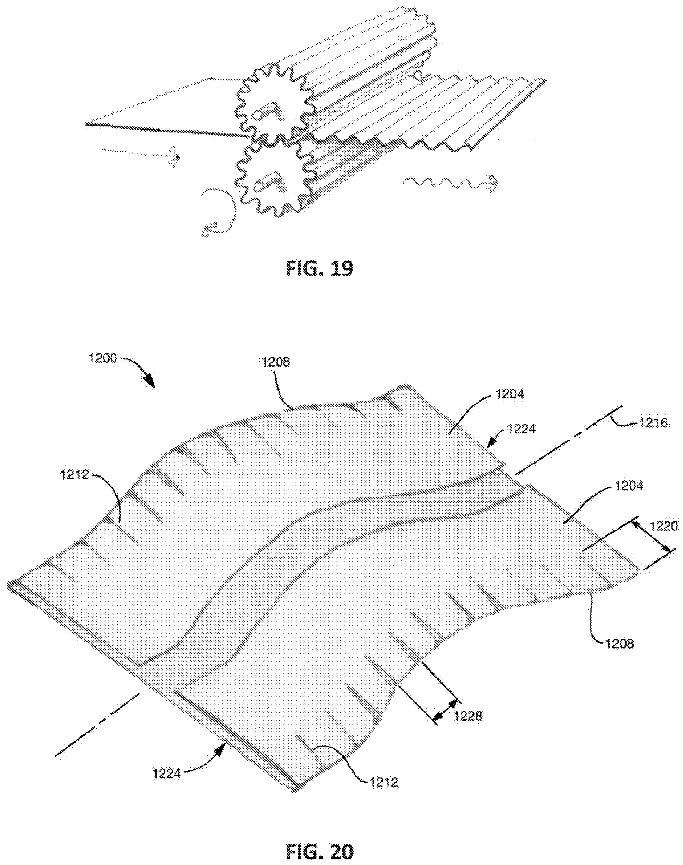

Certain of the present laminates were also softened by mechanical deformation processes. For example, laminates were temporarily corrugated by being passed between grooved/corrugated steel rollers, as illustrated in FIG. 19. As shown, two rollers are arranged such that the ridges or lands of one roller extend into the grooves of the other roller. In some examples, one or more sheets of the laminate are passed between the rolls without first being folded. In other examples, the laminate is folded into an MLC and the MLC is passed between the rollers. In either instance, the laminate or MLC may be passed between the rollers multiple times and/or in multiple directions (e.g., MD and CD).

TSA results did not indicate that samples mechanically deformed (e.g., temporarily corrugated) were any softer or smoother, but subjective assessments of softness by a human test panel indicated that the process meaningfully improved softness. This was because the corrugated rolls reduced the bending and shear strengths of the laminates, and these bulk mechanical properties were more of a factor in human panel testing than in the TSA measurement. Notably, the laminate is held in a planar configuration for the TSA measurement, providing primarily an indication of surface smoothness independent of bending and shear.

Measurements of the force required to shear the laminates were obtained using a Kawabata Evaluation System (KES). KES is used to make objective measurements of hand properties by measuring mechanical properties that correspond to the deformation of fabrics in hand manipulation. This testing indicated that the process also meaningfully improved the hand of the laminates and cores.

### 4. Slitting of Folded Multi-Layer Core

When folding the present laminates into MLCs, folded edges typically become more rigid than a single layer of laminate. However, adding slits through the edges of the MLCs can improve flexibility and improve conformability of the core when used inside a hygiene product and placed on a user. FIG. 20 depicts an example of such an MLC with slits. In particular, MLC 1200 includes folded lateral portions 1204 that define folded lateral edges 1208 of the core. As shown, the depicted configuration includes a plurality of slits 1212 through at least one layer of the laminate. In particular, slits 1212 extending from lateral edges 1208 toward a central longitudinal axis 1216 of the core. Each of slits 1212 has a length 1220 measured when the MLC is in its folded configuration which length may be different from than a length of the slit when the MLC is unfolded into a single layer of laminate. For example, a slit extending through three layers of laminate when the MLC is in its folded configuration may, when the laminate is in an unfolded state, comprise two slits-one with a length twice as large as the folded slit length, and another with a slit length roughly equal to the folded slit length. In the depicted configuration, slits 1212 are similar and corresponding pairs of slits on opposite sides of longitudinal axis 1216 are symmetrical with one another. In other configurations, the slits may be asymmetrical across access 1216 and/or may be of different lengths. For example, slits nearer the longitudinal ends of the MLC may have a folded slit length that is shorter than slits spaced inward from longitudinal ends 1224 of the MLC, for example to provide greater flexibility nearer a crotch region of the MLC. Further, in the depicted configuration, the slits are disposed at equal intervals or spaces 1228. In other configurations, the slits may be at uneven spaces; for example, slits nearer the longitudinal ends of the MLC may be farther apart than slits spaced inward from longitudinal ends 1224 of the MLC, for example to provide greater flexibility nearer a crotch region of the MLC. In general, the fewer the slits or the farther apart they are spaced, the less flexibility is imparted. The more slits added or the closer together they are spaced, the more flexibility is imparted. By changing the slit length and slit intervals, varying amounts of flexibility can be achieved.

## Claims

1. An absorbent core (200, 400, 500, 500a, 600, 600a, 1002) comprising an absorbent laminate (100, 100a, 100b) that comprises:
- a first laminate layer (102, 102a) comprising a tissue or nonwoven;
- a second laminate layer comprising a spunlace nonwoven (104, 104a); and
- an absorbent layer (106) positioned between the first and second laminate layers (102, 102a, 104, 104a), the absorbent layer (106) comprising adhesive and greater than about 90 percent by weight super absorbent polymer (108);
where at least one of the first and second laminate layers (102, 102a, 104, 104a) comprises a through-air dried tissue;
and wherein
lateral portions of the absorbent laminate (100, 100a, 100b) are folded inward toward a central longitudinal axis of the absorbent laminate (100, 100a, 100b) such that multiple layers of absorbent laminate (100, 100a, 100b) define a longitudinally folded absorbent core (200, 400, 500, 500a, 600, 600a, 1002);
**characterized in that**
(i) the second laminate layer (104, 104a) defines an outermost surface of the longitudinally folded absorbent core (200, 400, 500, 500a, 600, 600a, 1002); or **in that**
(ii) the absorbent layer is a first absorbent layer, and the absorbent laminate (100, 100a, 100b) further comprises:
- a third laminate layer comprising a spunlace nonwoven and disposed on an opposite side of the second laminate layer relative to the first laminate layer, wherein the third laminate layer defines an outermost surface of the longitudinally folded absorbent core (200, 400, 500, 500a, 600, 600a, 1002); and
- a second absorbent layer disposed between the second and third laminate layers, the second absorbent layer comprising adhesive and greater than about 90 percent by weight super absorbent polymer.

2. The absorbent core (200, 400, 500, 500a, 600, 600a, 1002) of the second characterizing alternative of claim 1, **characterized in that**
- the longitudinally folded absorbent core (200, 400, 500, 500a, 600, 600a, 1002) defines a longitudinal channel (C1, C2).

3. The absorbent core (200, 400, 500, 500a, 600, 600a, 1002) of the second characterizing alternative of claim 1, **characterized in that** the longitudinally folded absorbent core (200, 400, 500, 500a, 600, 600a, 1002) defines a longitudinal channel (C1, C2) and **in that** the channel (C1, C2) has a width of from 10 mm to 30 mm.

4. The absorbent core (200, 400, 500, 500a, 600, 600a, 1002) of any of claims 1-3, **characterized in that** the first laminate layer comprises tissue.

5. The absorbent core (200, 400, 500, 500a, 600, 600a, 1002) of any of claims 1-4, **characterized in that** the absorbent layer(s) each comprises from 40 grams per square meter (gsm) to 80 gsm of the super absorbent polymer (108).

6. The absorbent core (200, 400, 500, 500a, 600, 600a, 1002) of claim 5 and the second characterizing alternative of claim 1, **characterized in that** the total super absorbent polymer (108) content of all layers of the longitudinally folded absorbent core (200, 400, 500, 500a, 600, 600a, 1002) is from 200 gsm to 600 gsm or **in that** a basis weight of the second laminate layer is greater than a basis weight of the third laminate layer.

7. The absorbent core (200, 400, 500, 500a, 600, 600a, 1002) of any of claims 1-6, **characterized in that** the longitudinally folded absorbent core has three or more layers of the absorbent laminate.

8. The absorbent core (200, 400, 500, 500a, 600, 600a, 1002) of any of claims 1-7, **characterized in that** the absorbent laminate (100, 100a, 100b) has been mechanically softened by calendaring or temporary corrugating.

9. The absorbent core (200, 400, 500, 500a, 600, 600a, 1002) of any of claims 1-8, **characterized in that** the folding of the lateral portions of the laminate define folded lateral edges of the absorbent core (200, 400, 500, 500a, 600, 600a, 1002), and the absorbent core (200, 400, 500, 500a, 600, 600a, 1002) defines a plurality of slits through at least one layer of the laminate, the slits extending from the lateral edges toward the central longitudinal axis (C).

10. The absorbent core (200, 400, 500, 500a, 600, 600a, 1002) of any of claims 1-9, **characterized in that** the longitudinally folded absorbent core has a plurality of sheets of the absorbent laminate (100, 100a, 100b).

11. The absorbent core (200, 400, 500, 500a, 600, 600a, 1002) of any of claims 1-10, **characterized in that** the absorbent core (200, 400, 500, 500a, 600, 600a, 1002) comprises a surge core and a base core, and at least one of the surge and base cores is defined by the longitudinally folded absorbent laminate (100, 100a, 100b).

12. The absorbent core (200, 400, 500, 500a, 600, 600a, 1002) of any of claims 1-11, **characterized in that** the less than 3 percent of the weight of the super absorbent polymer (108) comes from particles that will not pass through a 500 µm screen.

13. A disposable absorbent article (1000) comprising :
- a body-facing topsheet (1001);
- a backsheet (1005); and
- an absorbent core (200, 400, 500, 500a, 600, 600a, 1002) of any of claims 1-12.

14. The disposable absorbent article of Claim 13, **characterized in that** the article further comprises an acquisition distribution layer (1106) positioned between the topsheet and the absorbent core (200, 400, 500, 500a, 600, 600a,1002), where a width of the acquisition distribution layer (1106) is at least 80% of a width of the longitudinally folded absorbent core (200, 400, 500, 500a, 600, 600a, 1002).

## Patentansprüche

1. Ein absorbierender Kern (200, 400, 500, 500a, 600, 600a, 1002), aufweisend ein absorbierendes Laminat (100, 100a, 100b), welches aufweist:
- eine erste Laminatschicht (102, 102a), die ein Gewebe oder Vlies aufweist;
- eine zweite Laminatschicht, die ein Spunlace-Vlies (104, 104a) aufweist; und
- eine absorbierende Schicht (106), die zwischen den ersten und zweiten Laminatschichten (102, 102a, 104, 104a) angeordnet ist, wobei die absorbierende Schicht (106) Klebstoff und mehr als etwa 90 Gewichtsprozent superabsorbierendes Polymer (108) aufweist;
wobei mindestens eine der ersten und zweiten Laminatschichten (102, 102a, 104, 104a) ein durchluftgetrocknetes Gewebe aufweist;
und wobei
seitliche Abschnitte des absorbierenden Laminats (100, 100a, 100b) nach innen in Richtung einer zentralen Längsachse des absorbierenden Laminats (100, 100a, 100b) gefaltet sind, so dass mehrere Schichten absorbierenden Laminats (100, 100a, 100b) einen in Längsrichtung gefalteten absorbierenden Kern (200, 400, 500, 500a, 600, 600a, 1002) definieren;
**dadurch gekennzeichnet, dass**
(i) die zweite Laminatschicht (104, 104a) eine äußerste Oberfläche des in Längsrichtung gefalteten absorbierenden Kerns (200, 400, 500, 500a, 600, 600a, 1002) definiert; oder dass
(ii) die absorbierende Schicht eine erste absorbierende Schicht ist, und das absorbierende Laminat (100, 100a, 100b) weiterhin aufweist:
- eine dritte Laminatschicht, die ein Spunlace-Vlies aufweist und auf einer mit Bezug zur ersten Laminatschicht gegenüberliegenden Seite der zweiten Laminatschicht angeordnet ist, wobei die dritte Laminatschicht eine äußerste Oberfläche des längs gefalteten absorbierenden Kerns (200, 400, 500, 500a, 600, 600a, 1002) definiert; und
- eine zweite absorbierende Schicht, die zwischen den zweiten und dritten Laminatschichten angeordnet ist, wobei die zweite absorbierende Schicht Klebstoff und mehr als 90 Gewichtsprozent superabsorbierendes Polymer aufweist.

2. Der absorbierende Kern (200, 400, 500, 500a, 600, 600a, 1002) nach der zweiten kennzeichnenden Alternative von Anspruch 1,
**dadurch gekennzeichnet, dass**
- der in Längsrichtung gefaltete absorbierende Kern (200, 400, 500, 500a, 600, 600a, 1002) einen Längskanal (C1, C2) definiert.

3. Der absorbierende Kern (200, 400, 500, 500a, 600, 600a, 1002) nach der zweiten kennzeichnenden Alternative von Anspruch 1,
**dadurch gekennzeichnet, dass**
der in Längsrichtung gefaltete absorbierende Kern (200, 400, 500, 500a, 600, 600a, 1002) einen Längskanal (C1, C2) definiert und dass der Längskanal (C1, C2) eine Breite von 10 mm bis 30 mm hat.

4. Der absorbierende Kern (200, 400, 500, 500a, 600, 600a, 1002) nach einem der Ansprüche 1 - 3,
**dadurch gekennzeichnet, dass** die erste Laminatschicht Gewebe aufweist.

5. Der absorbierende Kern (200, 400, 500, 500a, 600, 600a, 1002) nach einem der Ansprüche 1 - 4,
**dadurch gekennzeichnet, dass**
die absorbierende(n) Schicht(en) jeweils 40 Gramm pro Quadratmeter (g/m²) bis 80 g/m² superabsorbierendes Polymer (108) aufweisen.

6. Der absorbierende Kern (200, 400, 500, 500a, 600, 600a, 1002) nach Anspruch 5 und der zweiten kennzeichnenden Alternative von Anspruch 1, **dadurch gekennzeichnet, dass**
der Gesamtgehalt von superabsorbierendem Polymer (108) aller Schichten des in Längsrichtung gefalteten absorbierenden Kerns (200, 400, 500, 500a, 600, 600a, 1002) zwischen 200 g/m² und 600 g/m² liegt, oder dass ein Basisgewicht der zweiten Laminatschicht größer ist als ein Basisgewicht der dritten Laminatschicht.

7. Der absorbierende Kern (200, 400, 500, 500a, 600, 600a, 1002) nach einem der Ansprüche 1 - 6,
**dadurch gekennzeichnet, dass**
der in Längsrichtung gefaltete absorbierende Kern drei oder mehr Schichten des absorbierenden Laminats aufweist.

8. Der absorbierende Kern (200, 400, 500, 500a, 600, 600a, 1002) nach einem der Ansprüche 1 - 7,
**dadurch gekennzeichnet, dass**
das absorbierende Laminat durch Kalandrieren oder vorübergehendes Wellen mechanisch erweicht wurde.

9. Der absorbierende Kern (200, 400, 500, 500a, 600, 600a, 1002) nach einem der Ansprüche 1 - 8,
**dadurch gekennzeichnet, dass**
die Faltung der seitlichen Abschnitte des Laminats gefaltete Seitenkanten des absorbierenden Kerns (200, 400, 500, 500a, 600, 600a, 1002) definiert, und der absorbierende Kern (200, 400, 500, 500a, 600, 600a, 1002) mehrere Schlitze durch mindestens eine Schicht des Laminats definiert, wobei sich die Schlitze von den Seitenkanten in Richtung der zentralen Längsachse (C) erstrecken.

10. Der absorbierende Kern (200, 400, 500, 500a, 600, 600a, 1002) nach einem der Ansprüche 1 - 9,
**dadurch gekennzeichnet, dass**
der in Längsrichtung gefaltete absorbierende Kern mehrere Lagen des absorbierenden Laminats (100, 100a, 100b) aufweist.

11. Der absorbierende Kern (200, 400, 500, 500a, 600, 600a, 1002) nach einem der Ansprüche 1 - 10,
**dadurch gekennzeichnet, dass**
der absorbierende Kern (200, 400, 500, 500a, 600, 600a, 1002) einen Schwellkern und einen Basiskern aufweist, und mindestens einer von dem Schwellkern und dem Basiskern durch das in Längsrichtung gefaltete absorbierende Laminat (100, 1001a, 100b) definiert ist.

12. Der absorbierende Kern (200, 400, 500, 500a, 600, 600a, 1002) nach einem der Ansprüche 1 - 11,
**dadurch gekennzeichnet, dass**
weniger als 3 Prozent des Gewichts des superabsorbierenden Polymers (108) aus Partikeln stammen, die nicht durch ein 500-µm-Sieb passen.

13. Ein absorbierender Einwegartikel (1000) aufweisend:
- eine Körper-zugewandte obere Lage (1001);
- eine rückseitige Lage (1005); und
- einen absorbierenden Kern (200, 400, 500, 500a, 600, 600a, 1002) nach einem der Ansprüche 1 - 12.

14. Der absorbierende Einwegartikel nach Anspruch 13,
**dadurch gekennzeichnet, dass**
der Artikel weiterhin eine Aufnahmeverteilungsschicht (1106) aufweist, die zwischen der oberen Lage und dem absorbierenden Kern (200, 400, 500, 500a, 600, 600a, 1002) angeordnet ist, wobei eine Breite der Aufnahmeverteilungsschicht (1106) mindestens 80% einer Breite des in Längsrichtung gefalteten absorbierenden Kerns (200, 400, 500, 500a, 600, 600a, 1002) ist.

## Revendications

1. Âme absorbante (200, 400, 500, 500a, 600, 600a, 1002) comprenant un stratifié absorbant (100, 100a, 100b) qui comprend :
- une première couche de stratifié (102, 102a) comprenant un tissu ou un non-tissé ;
- une deuxième couche de stratifié comprenant un non-tissé hydrolié (104, 104a) ; et
- une couche absorbante (106) positionnée entre les première et deuxième couches de stratifié (102, 102a, 104, 104a), la couche absorbante (106) comprenant un adhésif et plus d'environ 90 pour cent en poids d'un polymère superabsorbant (108) ;
où au moins une parmi les première et deuxième couches de stratifié (102, 102a, 104, 104a) comprend un tissu séché à l'air ;
et dans laquelle
des portions latérales du stratifié absorbant (100, 100a, 100b) sont pliées vers l'intérieur vers un axe longitudinal central du stratifié absorbant (100, 100a, 100b) de telle sorte que de multiples couches de stratifié absorbant (100, 100a, 100b) définissent une âme absorbante pliée longitudinalement (200, 400, 500, 500a, 600, 600a, 1002) ;
**caractérisée en ce que**
(i) la deuxième couche de stratifié (104, 104a) définit une surface la plus externe de l'âme absorbante pliée longitudinalement (200, 400, 500, 500a, 600, 600a, 1002) ; ou **en ce que**
(ii) la couche absorbante est une première couche absorbante, et le stratifié absorbant (100, 100a, 100b) comprend en outre :
- une troisième couche de stratifié comprenant un non-tissé hydrolié et disposée sur un côté opposé de la deuxième couche de stratifié par rapport à la première couche de stratifié, dans laquelle la troisième couche de stratifié définit une surface la plus externe de l'âme absorbante pliée longitudinalement (200, 400, 500, 500a, 600, 600a, 1002) ; et
- une deuxième couche absorbante disposée entre les deuxième et troisième couches de stratifié, la deuxième couche absorbante comprenant un adhésif et plus d'environ 90 pour cent en poids d'un polymère superabsorbant.

2. Âme absorbante (200, 400, 500, 500a, 600, 600a, 1002) selon la deuxième variante caractérisante de la revendication 1, **caractérisée en ce que**
- l'âme absorbante pliée longitudinalement (200, 400, 500, 500a, 600, 600a, 1002) définit un canal longitudinal (C1, C2).

3. Âme absorbante (200, 400, 500, 500a, 600, 600a, 1002) selon la deuxième variante caractérisante de la revendication 1, **caractérisée en ce que** l'âme absorbante pliée longitudinalement (200, 400, 500, 500a, 600, 600a, 1002) définit un canal longitudinal (C1, C2) et **en ce que** le canal (C1, C2) a une largeur de 10 mm à 30 mm.

4. Âme absorbante (200, 400, 500, 500a, 600, 600a, 1002) selon l'une quelconque des revendications 1 à 3, **caractérisée en ce que** la première couche de stratifié comprend du tissu.

5. Âme absorbante (200, 400, 500, 500a, 600, 600a, 1002) selon l'une quelconque des revendications 1 à 4, **caractérisée en ce que** la (les) couche(s) absorbante(s) comprend (comprennent) chacune de 40 grammes par mètre carré (g/m²) à 80 g/m² du polymère superabsorbant (108).

6. Âme absorbante (200, 400, 500, 500a, 600, 600a, 1002) selon la revendication 5 et la deuxième variante caractérisante de la revendication 1, **caractérisée en ce que** la teneur totale en polymère superabsorbant (108) de toutes les couches de l'âme absorbante pliée longitudinalement (200, 400, 500, 500a, 600, 600a, 1002) est de 200 g/m² à 600 g/m² ou **en ce qu'**un grammage de la deuxième couche de stratifié est supérieur à un grammage de la troisième couche de stratifié.

7. Âme absorbante (200, 400, 500, 500a, 600, 600a, 1002) selon l'une quelconque des revendications 1 à 6, **caractérisée en ce que** l'âme absorbante pliée longitudinalement comporte trois couches ou plus du stratifié absorbant.

8. Âme absorbante (200, 400, 500, 500a, 600, 600a, 1002) selon l'une quelconque des revendications 1 à 7, **caractérisée en ce que** le stratifié absorbant (100, 100a, 100b) a été mécaniquement assoupli par calandrage ou ondulation temporaire.

9. Âme absorbante (200, 400, 500, 500a, 600, 600a, 1002) selon l'une quelconque des revendications 1 à 8, **caractérisée en ce que** le pliage des portions latérales du stratifié définit des bords latéraux pliés de l'âme absorbante (200, 400, 500, 500a, 600, 600a, 1002), et l'âme absorbante (200, 400, 500, 500a, 600, 600a, 1002) définit une pluralité de fentes à travers au moins une couche du stratifié, les fentes s'étendant des bords latéraux vers l'axe longitudinal central (C).

10. Âme absorbante (200, 400, 500, 500a, 600, 600a, 1002) selon l'une quelconque des revendications 1 à 9, **caractérisée en ce que** l'âme absorbante pliée longitudinalement comporte une pluralité de feuilles du stratifié absorbant (100, 100a, 100b).

11. Âme absorbante (200, 400, 500, 500a, 600, 600a, 1002) selon l'une quelconque des revendications 1 à 10, **caractérisée en ce que** l'âme absorbante (200, 400, 500, 500a, 600, 600a, 1002) comprend une âme de protection et une âme de base, et au moins une parmi les âmes de protection et de base est définie par le stratifié absorbant plié longitudinalement (100, 100a, 100b).

12. Âme absorbante (200, 400, 500, 500a, 600, 600a, 1002) selon l'une quelconque des revendications 1 à 11, **caractérisée en ce que** moins de 3 pour cent du poids du polymère superabsorbant (108) provient de particules qui ne passent pas à travers un tamis de 500 µm.

13. Article absorbant jetable (1000) comprenant :
- une couche supérieure (1001) faisant face au corps ;
- une enveloppe de couche (1005) ; et
- une âme absorbante (200, 400, 500, 500a, 600, 600a, 1002) de l'une quelconque des revendications 1 à 12.

14. Article absorbant jetable selon la revendication 13, **caractérisé en ce que** l'article comprend en outre une couche de distribution d'acquisition (1106) positionnée entre la couche supérieure et l'âme absorbante (200, 400, 500, 500a, 600, 600a, 1002), où une largeur de la couche de distribution d'acquisition (1106) est au moins 80 % d'une largeur de l'âme absorbante pliée longitudinalement (200, 400, 500, 500a, 600, 600a, 1002).
